(19) **Europäisches Patentamt**
**European Patent Office**
**Office européen des brevets**

(11) **EP 4 034 051 B1**

(12) **EUROPÄISCHE PATENTSCHRIFT**

(45) Veröffentlichungstag und Bekanntmachung des Hinweises auf die Patenterteilung:
**29.10.2025   Patentblatt 2025/44**

(21) Anmeldenummer: **20780584.7**

(22) Anmeldetag: **16.09.2020**

(51) Internationale Patentklassifikation (IPC):
***A61F 2/50*** *(2006.01)*      ***A61F 2/60*** *(2006.01)*

(52) Gemeinsame Patentklassifikation (CPC):
**A61F 2/7812;** A61F 2002/7837

(86) Internationale Anmeldenummer:
**PCT/EP2020/075830**

(87) Internationale Veröffentlichungsnummer:
**WO 2021/058346 (01.04.2021 Gazette 2021/13)**

(54) **PROTHESENÜBERZUG FÜR EINE PROTHESE, INSBESONDERE FÜR EINE BEINPROTHESE**

PROSTHESIS COVER FOR A PROSTHESIS, PARTICULARLY FOR A PROSTHETIC LEG

REVÊTEMENT DE PROTHÈSE POUR UNE PROTHÈSE, EN PARTICULIER POUR UNE JAMBE PROTHÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.09.2019   DE 102019125870**
**27.07.2020   DE 102020119762**

(43) Veröffentlichungstag der Anmeldung:
**03.08.2022   Patentblatt 2022/31**

(73) Patentinhaber: **Romedis GmbH**
**83115 Neubeuern (DE)**

(72) Erfinder: **RADSPIELER, Andreas**
**83115 Neubeuern (DE)**

(74) Vertreter: **Pfenning, Meinig & Partner mbB**
**Patent- und Rechtsanwälte**
**Theresienhöhe 11a**
**80339 München (DE)**

(56) Entgegenhaltungen:
WO-A1-2019/110543       DE-A1- 102016 113 590
GB-A- 2 067 074        US-A1- 2007 150 069
US-A1- 2015 081 038

EP 4 034 051 B1

**Beschreibung**

Die vorliegende Erfindung betrifft einen Prothesenüberzug mit einem geschlossenen Querschnitt, d. h. zum Hineinschlüpfen oder zum Hineinsteigen mit der Extremität, etwa dem Bein, gemäß Anspruch 1 oder 11, eine Prothese gemäß Anspruch 9 und ein Set gemäß Anspruch 10.

Aus der Praxis sind Prothesenüberzüge bekannt, die auf der Haut und insbesondere ein Gelenk oder eine Prothese eines Patienten überbrückend angelegt werden. So offenbart die WO 2019/110543 A1 z. B. einen Prothesenüberzug mit einem geschlossenen Querschnitt.

Eine Aufgabe der vorliegenden Erfindung ist es, einen weiteren Prothesenüberzug und eine Prothese vorzuschlagen.

Die erfindungsgemäße Aufgabe wird durch einen Prothesenüberzug mit den Merkmalen des Anspruchs 1, durch eine Prothese mit den Merkmalen des Anspruchs 8 und durch ein Set mit den Merkmalen des Anspruchs 9 gelöst.

Hierbei weist der Prothesenüberzug entlang seiner Längserstreckung (oder Längsrichtung) wenigstens oder genau einen ersten, zweiten und dritten Abschnitt auf oder besteht hieraus. Der zweite Abschnitt ist dabei in Längsrichtung zwischen dem ersten und dem dritten Abschnitt angeordnet.

Der zweite Abschnitt besteht, z. B. in einem zweiten Teilabschnitt hiervon, optional aus einem Material mit einem größeren Elastizitätsmodul (kurz: E-Modul, alternativ Youngscher Modul, alternativ definiert als Quotient aus Spannung und Dehnung ($\sigma/\varepsilon$)) als der erste und/oder der dritte Abschnitt oder die Materialien des ersten und/oder des dritten Abschnitts, oder weist optional ein solches Material auf.

Ergänzend oder alternativ zu den vorstehend genannten unterschiedlichen Elastizitätsmodulen weist der zweite Abschnitt einen größeren Umfang und/oder Durchmesser auf als der erste Abschnitt und auch als der dritte Abschnitt.

Der Prothesenüberzug dient dazu, eine Extremitätenprothese, insbesondere eine Beinprothese, in ihrem bestimmungsgemäßen Gebrauch zumindest abschnittsweise zu bedecken. Bestimmungsgemäß kann der Prothesenüberzug einerseits auf dem Körpergliedstumpf direkt oder durch wenigstens eine Materialschicht getrennt aufliegen und andererseits auf einem Abschnitt der Prothese, etwa einer Kosmetik, direkt oder durch wenigstens eine Materialschicht getrennt aufliegen. Dabei kann der Prothesenüberzug ein künstliches Gelenk der Prothese überbrücken, bedecken oder umschließen.

Die erfindungsgemäße Prothese, die insbesondere als Beinprothese oder Knieprothese ausgestaltet sein kann, ist zumindest teilweise durch einen erfindungsgemäßen Prothesenüberzug bedeckt oder überdeckt.

Das erfindungsgemäße Set weist eine Prothese und einen erfindungsgemäßen Prothesenüberzug auf.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen können eines oder mehrere der vorstehend oder im Folgenden genannten Merkmale aufweisen. Dabei können die hierin genannten Merkmale in beliebiger Kombination Gegenstand von erfindungsgemäßen Ausführungsformen sein, sofern der Fachmann eine konkrete Kombination nicht als technisch unmöglich erkennt.

Erfindungsgemäße Ausführungsformen sind ferner Gegenstand der Unteransprüche und Ausführungsformen.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin von einer Ausführungsform die Rede ist, so handelt es sich um eine beispielhafte, erfindungsgemäße Ausführungsform.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

In manchen Ausführungsformen weist der zweite Abschnitt zumindest einen ersten Teilabschnitt auf, welcher schlauchförmig ausgestaltet ist und mit dem ersten und/oder dem dritten Abschnitt, die ebenfalls schlauchförmig ausgestaltet sein können, vorzugweise luftdicht verbunden ist.

In bestimmten Ausführungsformen weist der zweite Abschnitt zumindest einen zweiten Teilabschnitt auf, welcher bandförmig zwischen dem ersten und dem dritten Abschnitt verläuft, insbesondere in einer Längsrichtung des Prothesenüberzugs.

In manchen Ausführungsformen weist der zweite Abschnitt den hierin als ersten und/oder den hierin als zweiten bezeichneten Teilabschnitt auf, oder nur einen dieser beiden, oder besteht aus einem oder beiden dieser Teilabschnitte.

In bestimmten Ausführungsformen wurden der erste Teilabschnitt und der zweite Teilabschnitt getrennt voneinander und/oder aus unterschiedlichen Materialien gefertigt (oder weisen solche auf) bevor sie Teil des zweiten Abschnitts oder des Prothesenüberzugs wurden.

In einigen Ausführungsformen ist der zweite Abschnitt zumindest in seinem ersten Teilabschnitt in wenigstens einem Zustand hiervon gefaltet oder aufgeworfen oder weist anderweitig eine Materialreserve auf.

Bei einem Prothesenüberzug, wie hierin verwendet, kann es sich auch um eine(n) Dichtüberzug, Schutzhülle, Vakuum(knie)bandage, Manschette, Prothesenmanschette, Prothesenliner handeln. Das Austauschen dieser Begriffe gegeneinander führt in jedem Fall zu Merkmalskombinationen, die ebenfalls von der vorliegenden Erfindung umfasst sind.

In einigen Ausführungsformen ist der Prothesenüberzug kein Liner. Er ist optional nicht bestimmt, um unter einem Prothesenschaft getragen zu werden. Er ist optional bestimmt, um über der Prothese getragen zu werden.

In manchen Ausführungsformen weist der zweite Abschnitt zumindest einen ersten Teilabschnitt auf, welcher in zumindest einem Zustand des Prothesenüberzugs gedoppelt ist oder in Falten, Wellen und/oder Auf- oder Verwerfungen liegt, oder auf diese oder andere Weise eine Materialreserve bietet, vorzugweise in einem Gebrauchszustand (also angezogen, d. h. am Körper des Patienten oder Trägers verwendet) und/oder in wenigstens einem Nicht-Gebrauchszustand (also z. B. in seiner Versandverpackung, ausgepackt auf dem Tisch liegend, usw.).

In anderen Ausführungsformen weist der Prothesenüberzug und insbesondere der zweite Abschnitt keinen gedoppelten Teilabschnitt und/oder keinen Faltenbalg auf.

Unter einem gedoppelten Teilabschnitt kann ein Teilabschnitt verstanden werden, bei dem Material, welches den Teilabschnitt bildet oder an seiner Bildung beteiligt ist, also z. B. dessen Wandung oder Schicht oder Schichtdicke, wenigstens abschnittsweise zumindest zweilagig übereinander oder nebeneinander liegt. Dabei liegen zwei Lagen des Materials, aus welchem die Wandung des Teilabschnitts besteht, aufeinander oder nebeneinander, was z. B. mit einer Faltenbildung verglichen werden kann. Ein Entfalten des Teilabschnitts könnte zu einer größeren Länge (z. B. in einer Richtung quer zum Verlauf der Falte) des Teilabschnitts führen. Die Doppelung würde dabei mehr oder weniger aufgegeben werden und sich spätestens bei einem vollständigen Entfalten des Teilabschnitts vollständig auflösen. Bei vollständiger Entfaltung könnte, je nach Ausgestaltung, der Teilabschnitt nur mehr einlagig vorliegen.

Ein Beispiel einer Doppelung ist von der Ziehharmonika bekannt, welche einen Balg als eine der wesentlichen Komponenten aufweist. Dabei erstreckt sich die Breite des Bereichs, in welchem die Lagen des Materials in Falten aufeinander liegen, üblicherweise quer zur Längsrichtung des Balgs. Optional verläuft die Breite, in welchem die Lagen des Materials in Falten aufeinander liegen, ganz, überwiegend oder im Wesentlichen in Längsrichtung und/oder in Querrichtung oder Umfangsrichtung des Teilabschnitts oder des Prothesenüberzugs.

Unter einer Lage der Wandung ist in manchen Ausführungsformen die gesamte Dicke oder Stärke der, zum Beispiel textilen, Wandung zu verstehen. Faltet man die Wandung, so liegt sie in Lagen aufeinander oder nebeneinander. Lagen, wie hierin verwendet, sind in manchen Ausführungsformen nicht mit Schichten zu verwechseln. Eine Wandung kann mehrere Schichten aufweisen, z. B. eine innere Schicht, die aus einem besonders hautfreundlichen Material besteht oder ein solches aufweist, und einem besonders wasser- oder luftdichten Material, das als äußere Schicht auf der inneren Schicht aufliegt. Dabei bilden die innere Schicht und die äußere Schicht bei vollständig glattgestrichener Wandung gemeinsam nur eine Lage. Erst wenn man die Wandung faltet, so dass sich - von innen nach außen - eine innere Schicht, eine äußere Schicht, eine weitere äußere Schicht, eine innere Schicht und schließlich eine äußere Schicht aneinander anschließen, würde man hierin davon reden, dass die Wandung in zwei Lagen oder gedoppelt vorliegt.

Vorstehende Ausführungen zur inneren und äußeren Schicht können in einigen Ausführungsformen auf die innere bzw. äußere Seite übertragen werden.

In manchen Ausführungsformen ist die Wandung des ersten, zweiten und/oder dritten Abschnitts oder von Teilabschnitten hiervon, etwa dem hierin beschriebenen ersten und/oder dem zweiten hierin beschriebenen Teilabschnitt des zweiten Abschnitts, aus genau einer Schicht gefertigt.

In einigen Ausführungsformen weist der zweite Abschnitt zumindest einen ersten Teilabschnitt auf. Auf ihn können die hierin zu einem ersten Teilabschnitt gemachten Beschreibungen in beliebiger Kombination zutreffen.

In manchen Ausführungsformen weist der zweite Abschnitt zumindest einen zweiten Teilabschnitt auf. Auf ihn können die hierin zu einem zweiten Teilabschnitt gemachten Beschreibungen in beliebiger Kombination zutreffen.

In einigen Ausführungsformen weist der zweite Abschnitt zumindest einen ersten Teilabschnitt auf, welcher in zumindest einem Zustand des Prothesenüberzugs oder in jedem Gebrauchszustand des Prothesenüberzugs aufgeworfen ist.

In einigen Ausführungsformen weist der zweite Abschnitt zumindest einen ersten Teilabschnitt auf, welcher in zumindest einem Zustand, etwa im Gebrauchszustand, des Prothesenüberzugs eine Materialreserve aufweist.

Der Begriff der "Materialreserve" eines Abschnitts kann hierin bedeuten, dass der Abschnitt Material oder potentielle Länge aufweist, welche ihn gedoppelt, in Falten, Wellen und/oder Verwerfungen liegt lässt, insbesondere z. B.

nicht gestreckt liegen lässt, etwa in einer Längsrichtung des Abschnitts und/oder in einer Quer- oder einer Umfangsrichtung des Abschnitts. So würde man von einer Ziehharmonika (ein Musikinstrument mit einem Balg, der durch das abwechselnde Zusammendrücken und Auseinanderziehen des Balgs Luftströme erzeugt, die Zungen in Schwingung bringen und somit Töne erzeugen), wenn ihr Balg nicht vollkommen auseinander gezogen ist, im Sinne des Begriffs der "Materialreserve", wie er hierin verwendet wird, sagen, dass sie oder ihr Balg eine Materialreserve aufweisen, da man diesen noch weiter auseinanderziehen und damit längen oder strecken könnte. Es ist offensichtlich, dass der Balg in den meisten Zuständen, die er einnehmen kann, eine solche Materialreserve aufweist. Nur wenn er vollständig auseinandergezogen ist, so dass er ohne zerstörend zu wirken nicht weiter auseinandergezogen werden könnte, hat er eine solche Materialreserve nicht mehr.

Eine Streckung derart, dass die Materialreserve des ersten Teilabschnitts in Längsrichtung des Prothesenüberzugs aufgebraucht werden würde, der erste Teilabschnitt somit vollständig gestreckt wäre, tritt bei Vorsehen des zweiten Teilabschnitts jedoch in manchen Ausführungsformen während des Tragens des Prothesenüberzugs nicht ein. Dies wird durch den in Längsrichtung des Prothesenüberzugs kürzeren zweiten Teilabschnitt verhindert.

Der Begriff der "Materialreserve" kann in einigen Ausführungsformen durch "Längenreserve" und/oder "Umfangsreserve" ersetzt werden.

Wenn hierin die Rede davon ist, dass ein Abschnitt oder ein Teilabschnitt eine Materialreserve oder Längenreserve aufweist oder nicht, so kann sich dies auf einen Zustand beziehen, in welcher der Prothesenüberzug maximal gestreckt wird, etwa per Hand, mit leichter oder mittlerer Kraft, mit welcher der Prothesenüberzug in seiner Längsrichtung auseinandergezogen oder gestreckt wird, wie dies z. B. beim bestimmungsgemäßen Anziehen oder Anlegen des Prothesenüberzugs erfolgen könnte.

Wenn hierin die Rede davon ist, dass ein Abschnitt oder ein Teilabschnitt eine Materialreserve oder Längenreserve aufweist oder nicht, so kann sich dies in einigen Ausführungsformen auf eine Materialreserve in der Breite oder in der Abwicklung beziehen. Materialreserven können in manchen Ausführungsformen sowohl in der Länge als auch in der Breite sowie in einer Umfangsrichtung des Prothesenüberzugs vorgesehen sein.

Der Begriff der "Materialreserve" kann in einigen Ausführungsformen somit alternativ oder ergänzend durch "Reserve in der Breite oder bei der Abwicklung" ersetzt werden.

In bestimmten Ausführungsformen ist das Material des ersten Teilabschnitts und/oder das Material des zweiten Teilabschnitts nicht in Längsrichtung des Prothesenüberzugs dehnbar.

In einigen Ausführungsformen ist das Material des ersten Teilabschnitts in Längsrichtung und/oder Umfangsrichtung des Prothesenüberzugs dehnbar. Damit kann sich der erste Teilabschnitt nach wenigen Schritten mit der Prothese bereits an das Bein/die Prothese anlegen. Dies geschieht, da der erste Teilabschnitt dank des zweiten Teilabschnitts keine Kräfte in der Längsrichtung des Prothesenabschnitts übertragen muss, etwa beim Überziehen des Prothesenüberzugs, ohne radialen Druck oder nennenswerten radialen Druck und damit für den Patienten auf sehr angenehme Weise.

In manchen Ausführungsformen weist der zweite Abschnitt zumindest einen ersten Teilabschnitt auf, welcher in zumindest einem Zustand des Prothesenüberzugs lamellenartig ist.

In einigen Ausführungsformen weist der zweite Abschnitt zumindest einen ersten Teilabschnitt auf, welcher in zumindest einem Zustand des Prothesenüberzugs vollständig oder zumindest abschnittsweise mehrlagig ist.

In manchen Ausführungsformen weist der zweite Abschnitt zumindest einen ersten Bereich auf, welcher in zumindest einem Zustand des Prothesenüberzugs vollständig oder zumindest abschnittsweise mehrlagig ist, während ein zweiter Bereich, welcher größer als der erste Bereich ist, nicht mehrlagig ist.

In weiteren Ausführungsformen weist der zweite Abschnitt wenigstens eine in Längsrichtung oder zumindest auch in Längsrichtung des Prothesenüberzugs oder des zweiten Abschnitts verlaufende Struktur auf, die - vorzugsweise für sich genommen oder in sich - optional nicht gefaltet, nicht gedoppelt, nicht aufgeworfen, nicht lamellenartig oder nicht mehrlagig ist.

In einigen Ausführungsformen ist diese Struktur die hierin als zweiter Teilabschnitt bezeichnete Komponente des zweiten Abschnitts. Sie kann zwischen erstem und drittem Abschnitt liegen, mit diesen jeweils verbunden sein und/oder angeordnet sein, und kann ein Dehnen des Prothesenüberzugs oder seines zweiten Abschnitts beim Versuch, den Prothesenüberzug in dessen Längsrichtung maximal zu dehnen, bereits begrenzen oder beenden, wenn der erste Teilabschnitt noch weiter dehnbar wäre. Der zweite Teilabschnitt kann somit vorzugsweise das Maß, in welchem der erste und der dritte Abschnitt beim Dehnen oder Strecken des Prothesenüberzugs, etwa wenn der Patient den Prothesenüberzug überzieht oder anzieht, sich voneinander entfernen könnten, maßgeblich begrenzen oder bestimmen. Ein weiteres Dehnen des Prothesenüberzugs oder des zweiten Abschnitts, welches theoretisch noch möglich wäre, da der erste Teilabschnitt des zweiten Abschnitts in Längsrichtung noch Materialreserve hat oder noch weiter dehnbar wäre bei den angewandten Zugkräften, welche z. B. beim Überziehen des Prothesenüberzugs auf die Prothese durch den Patienten wirken, kann durch den zweiten Teilabschnitt in manchen Ausführungsformen somit verhindert werden.

In manchen Ausführungsformen ist oder umfasst diese Struktur oder der zweite Teilabschnitt wenigstens ein Band oder wenigstens eine bandartige Struktur, die eine Breite zwischen 0,5 cm und 10 cm, vorzugsweise zwischen 1 cm und 7 cm, ganz besonders bevorzugt zwischen 1 cm und 5 cm, zwischen 1,5 cm und 5 cm oder zwischen 1 cm und 3 cm hat.

In einigen Ausführungsformen ist oder umfasst diese Struktur oder der zweite Teilabschnitt wenigstens ein Band oder wenigstens eine bandartige Struktur, die eine Länge aufweist, welche dem Abstand zwischen einander zugewandten Rändern des ersten Abschnitts einerseits und des dritten Abschnitts andererseits (z. B. bei maximaler Streckung des Prothesenüberzugs) entspricht, oder zwischen 80 % und 120 % hiervon beträgt, z. B. in einem Bereich des Prothesenüberzugs, in welchem auch die bandartige Struktur oder der zweite Teilabschnitt angeordnet ist.

In einigen Ausführungsformen ist der E-Modul des Materials dieser Struktur oder des zweiten Teilabschnitts größer als der E-Modul des ersten Abschnitts und/oder des dritten Abschnitts, und/oder kleiner oder größer als der E-Modul des Materials des ersten Teilabschnitts des zweiten Abschnitts, vorzugsweise zumindest in einer gemeinsamen Richtung, etwa jeweils in der Längsrichtung des Prothesenüberzugs.

In manchen Ausführungsformen verlaufen alle oder manche der Lamellen, Falten, Verwerfungen, mehrlagigen Abschnitte oder Doppelungen in der Umfangsrichtung, vorzugsweise nicht in der Längsrichtung, des Prothesenüberzugs oder des zweiten Abschnitts.

In einigen Ausführungsformen weist der zweite Abschnitt zumindest einen ersten Teilabschnitt auf, welcher die Materialreserve in Längsrichtung des zweiten Abschnitts oder des Prothesenüberzugs aufweist.

In einigen Ausführungsformen weist der erste, zweite und/oder dritte Abschnitt des Prothesenüberzugs ein luftdichtes Material auf oder besteht hieraus.

In manchen Ausführungsformen sind der erste, zweite und/oder dritte Abschnitt des Prothesenüberzugs miteinander luftdicht verbunden, etwa durch Verkleben oder Textilschweißen.

In bestimmten Ausführungsformen weist der erste Teilabschnitt des zweiten Abschnitts des Prothesenüberzugs ein luftdichtes Material auf oder besteht hieraus. Dieses Material kann sich optional entlang des gesamten Umfangs des zweiten Abschnitts erstrecken.

In manchen Ausführungsformen sind der erste Teilabschnitt und/oder der zweite Teilabschnitt des zweiten Abschnitts des Prothesenüberzugs nicht elastisch.

In manchen Ausführungsformen ist der erste Teilabschnitt, nicht aber auch der zweite Teilabschnitt des zweiten Abschnitts des Prothesenüberzugs elastisch.

In einigen Ausführungsformen sind der erste und/oder der dritte Abschnitt des Prothesenüberzugs elastisch.

In manchen Ausführungsformen bedeutet "nicht-dehnbar" oder "nicht-elastisch", dass der Elastizitätsmodul, abgekürzt als E-Modul, des betreffenden Teilabschnitts wenigstens oberhalb 200 N/mm$^2$, bevorzugt oberhalb 700 N/mm$^2$, vorzugsweise oberhalb 1000 N/mm$^2$, besonders bevorzugt oberhalb 2000 N/mm$^2$, liegt.

In bestimmten Ausführungsformen bedeutet "nicht-dehnbar" oder "nicht-elastisch", dass eine Dehnung des betreffenden Teilabschnitts nicht mehr als 20 %, bevorzugt nicht mehr als 10 %, vorzugsweise nicht mehr als 5 %, besonders bevorzugt nicht mehr als 2 % seiner Länge betragen darf, bevor der Teilabschnitt reißt.

In manchen Ausführungsformen hat der betreffende Teilabschnitt oder Fasern hiervon einen E-Modul wie Nylon.

In bestimmten Ausführungsformen bedeutet "dehnbar" oder "elastisch" das Gegenteil von "nicht-dehnbar" bzw. "nicht-elastisch". Ein Stoff oder Körper ist in diesen Ausführungsformen somit entweder dehnbar oder aber nicht-dehnbar bzw. elastisch oder aber nicht-elastisch.

In manchen Ausführungsformen weist der zweite Abschnitt des Prothesenüberzugs in wenigstens einem Teilabschnitt hiervon (z. B. dem ersten Teilabschnitt) eine, z. B. textile, erhabene Struktur auf seiner Innenseite auf, also auf der, der Körperextremität beim Tragen des Prothesenüberzugs zugewandten, Seite des Prothesenüberzugs bzw. des zweiten Abschnitts.

Der erste Teilabschnitt kann optional aus einem Kunststoff (PP, PE oder anderem), einem faserverstärkten Kunststoff, einem Verbundmaterial, einem laminierten Material, einem beschichteten Material, einem beschichteten Textil oder einem Gewebe hergestellt sein oder ein solches Material aufweisen.

Der erste Teilabschnitt ist vorzugsweise luftdicht oder aus luftdichtem Material oder weist ein solches auf.

Der zweite Teilabschnitt kann aus einem Kunststoff (PP, PE oder anderem), einem faserverstärkten Kunststoff, einem Verbundmaterial, einem laminierten Material, einem Gewebe hergestellt sein oder ein solches Material aufweisen.

Der erste Teilabschnitt und der zweite Teilabschnitt unterscheiden sich optional hinsichtlich ihres Materials.

Der erste Abschnitt und/oder der dritte Abschnitt können aus Silikon, Elastomer, Silikon-Elastomermaterial, insbesondere einem kontinuierlichen, ausgehärteten Silikon-Elastomermaterial hergestellt sein oder ein solches Material aufweisen.

In bestimmten Ausführungsformen bietet der Prothesenüberzug oder der zweite Abschnitt eine Materialreserve in der Längsrichtung des Prothesenüberzugs. Die Materialreserve kann sich optional auf den - falls vorgesehen - ersten Teilabschnitt des zweiten Abschnitts beziehen. Es kann vorgesehen sein, dass der zweite Teilabschnitt des zweiten Abschnitts - falls vorgesehen - keine Materialreserve hat.

Die Materialreserve kann - z. B. bei Vergleich des vollkommen gefalteten Zustands mit dem vollständig entfalteten Zustand - 10 %, 20 %, 30 %, 40 % oder mehr ausmachen. So kann sich ein Prothesenüberzug oder deren zweiter Abschnitt durch Entfalten, z. B. nach Trennen des zweiten Abschnitts vom ersten und/oder dritten Abschnitt, beispielsweise um einen der vorstehend genannten Prozentwerte, oder Zwischenwerte hiervon, durch Entfalten verlängern lassen. Das Entfalten geht dabei vorzugsweise nicht oder im Wesentlichen nicht auf elastische Materialeigenschaften zurück, sondern auf das Auseinanderfalten oder Glattziehen z. B. von Lamellen, Falten, Wellen, Doppelungen, usw.

In einigen Ausführungsformen weist der Prothesenüberzug kein geschlossenes Ende auf und kann somit als schlauchförmig bezeichnet werden. In anderen weist er ein geschlossenes Ende auf und kann somit als sackförmig bezeichnet werden.

In manchen Ausführungsformen weist der Prothesenüberzug einen oder mehrere elastifizierte Stoffe auf, in anderen nicht, was insbesondere für den zweiten Abschnitt oder den ersten Teilabschnitt des zweiten Abschnitts zutreffen kann.

In einigen Ausführungsformen weist nicht jedes Element oder nicht jeder Abschnitt des Prothesenüberzugs elastifizierte Stoffe auf, was insbesondere für den zweiten Teilabschnitt des zweiten Abschnitts zutreffen kann.

In manchen Ausführungsformen weist nicht jedes Element oder nicht jeder Abschnitt des Prothesenüberzugs Stoffe oder Materialien auf, die eine größere Elastizität, E-Modul oder elastische Steifigkeit in der einen Richtung als in der orthogonal zu dieser einen Richtung verlaufenden anderen Richtung besitzen.

In manchen Ausführungsformen weist der Prothesenüberzug Stoffe oder Materialien auf, die eine größere Elastizität, E-Modul oder elastische Steifigkeit in einer oder in der einen Richtung als in der orthogonal zu dieser Richtung verlaufenden anderen Richtung besitzen. Dies kann insbesondere auf den ersten Teilabschnitt des zweiten Abschnitts zutreffen.

In einigen Ausführungsformen weist der Prothesenüberzug Stoffe oder Materialien auf, die ein größeres E-Modul in einer oder in der einen Richtung als in der orthogonal zu dieser Richtung verlaufenden anderen Richtung besitzen. Dies kann insbesondere auf den ersten Teilabschnitt des zweiten Abschnitts zutreffen.

In einigen Ausführungsformen ist die Innenfläche des Prothesenüberzugs nicht und/oder nicht vollständig mit einem Silikon-Elastomermaterial, insbesondere nicht mit einem kontinuierlichen, ausgehärteten Silikon-Elastomermaterial bedeckt. In manchen Ausführungsformen sind der ersten und/oder der dritte Abschnitt aus Silikon oder weisen Silikon auf. In einigen Ausführungsformen weist der zweite Abschnitt kein Silikon auf oder besteht nicht hieraus.

In manchen Ausführungsformen weist der zweite Abschnitt des Prothesenüberzugs kein Gel auf.

In einigen Ausführungsformen weist der Prothesenüberzug kein Gel auf.

In manchen Ausführungsformen ist der Prothesenüberzug keine Gelenkbandage zum Überziehen, besteht nicht aus einem elastischen Schlauch mit einer Vorderseite und einer dieser gegenüberliegenden Beugeseite, wobei die Beugeseite zumindest bereichsweise zweilagig ausgebildet ist, wobei der elastische Schlauch aus einem Vorder- und einem Rückteil zusammengefügt ist, wobei das Rückteil die Beugeseite bildet.

In einigen Ausführungsformen weist der Prothesenüberzug auf seiner Beugeseite keine flachgestrickten Bereiche auf, zwischen denen ein schlauchartiges Rundgestrick eingestrickt ist, welches im Beugebereich angeordnet ist.

In manchen Ausführungsformen ist der Prothesenüberzug keine Gelenkbandage mit einem schlauchförmigen Grundkörper aus einem elastischen Textilmaterial mit einem Einsatz im Beugebereich, wobei die Elastizität des Einsatzes größer als die des Grundkörpers ist.

In einigen Ausführungsformen weist der Prothesenüberzug keinen Einsatz mit zwei Bereichen mit unterschiedlicher Elastizität auf, deren Einzelelastizitäten größer als die des Grundkörpers sind.

In manchen Ausführungsformen ist der Prothesenüberzug kein aus textilen Fäden hergestelltes Flächengebilde, mit einer zumindest auf einer Seite ausgebildete Querwellenstruktur, welche durch eine eingearbeitete oder unter einer Deckstruktur liegende elastische Fadenanordnung, welche in vorbestimmten Abständen mit der Deckstruktur verbunden ist, elastisch vorgespannt und stabilisiert ist.

In einigen Ausführungsformen weist der Prothesenüberzug kein Textillaminat mit einer elastischen Textilbasis und einer darauf angeordneten Kunststoffbeschichtung auf, die dünner als die Textilbasis ist und in die eine Perforation zur Einstellung einer angepassten Elastizität des Textillaminats eingebracht ist.

In manchen Ausführungsformen weist der Prothesenüberzug keine Bestückungen auf. Hierunter zählen insbesondere aufgebrachte, etwa aufgeklebte oder aufgenähte Flecken, Patches, Pads, Kissen. In bestimmten Ausführungsformen weist der Prothesenüberzug jedoch ein Dichtband auf, welches in Längsrichtung des zweiten Abschnitts und insbesondere des ersten Teilabschnitts hiervon verlaufende Stoßränder, vorzugsweise innenseitig, verklebt und/oder abdichtet.

In einigen Ausführungsformen ist der Prothesenüberzug keine Kompressionsbandage, insbesondere nicht zum Tragen auf oder über dem gesunden Knie, vor allem, wenn durch einen mittleren Abschnitt des Prothesenüberzugs Druck auf das Knie ausgeübt werden soll.

In manchen Ausführungsformen weist der Prothesenüberzug keine Strick- oder Strichorientierungen auf, insbesondere keine Abschnitte mit unterschiedlichen Strick- oder Strichorientierungen.

In einigen Ausführungsformen weist der Prothesenüberzug auf seiner Innenseite und/oder auf seiner Außenseite keine Vorsprünge auf.

In manchen Ausführungsformen weist der Prothesenüberzug auf seiner Innenseite und/oder auf seiner Außenseite kein Material auf, welches vorgesehen ist, um eine Bewegung des Prothesenüberzugs auf der Haut oder einer über der Haut angeordneten Schicht zu verhindern.

In einigen Ausführungsformen weist der Prothesenüberzug in seiner Längsrichtung drei oder genau drei Abschnitte (z. B. den ersten, zweiten und dritten Abschnitt) auf, die mit Verklebungen, Nähten oder auf andere Weise zum Erzeugen einer schlauchförmigen Struktur miteinander verbunden sind. Derartige Verklebungen, Nähte oder dergleichen verlaufen optional nur in Umfangsrichtung, optional nicht in Längsrichtung des Prothesenüberzugs, wobei aber wenigstens einer der drei Abschnitte seinerseits zu einer ring- oder schlauchförmigen Struktur verklebt, vernäht oder auf sonstige Weise mit sich selbst verbunden ist. Wenigstens einer dieser Abschnitte kann, z. B. an seiner Außenseite oder Innenseite, eine Struktur aufweisen, wie sie hierin als zweiter Teilabschnitt des zweiten Abschnitts beschrieben ist.

Wenn hierin die Rede davon ist, dass der erste, zweite und dritte Abschnitt aufeinander folgen, z. B. indem sie miteinander verklebt, vernäht oder auf andere Weise zum Erzeugen einer schlauchförmigen Struktur miteinander verbunden sind, so kann dies z. B. an der Außenhaut oder äußeren Schicht des Prothesenüberzugs der Fall sein, und die drei Abschnitte können z. B. mit Blick von außen auf den Prothesenüberzug unterscheidbar sein. Dabei ist es für die Gliederung in ersten, zweiten und dritten Abschnitt unschädlich, dass z. B. auf einer Innenseite des Prothesenüberzugs ein Gel oder einer Stoff, der den Tragekomfort insbesondere des ersten und dritten Abschnitts erhöhen kann, sich bis z. B. über eine Naht zwischen erstem und zweitem oder drittem und zweitem Abschnitt erstreckt, was den Tragekomfort durch Bedecken der Naht weiter erhöhen kann.

In anderen Ausführungsformen erstreckt sich eine Außenhaut derart über den ersten, zweiten und/oder dritten Abschnitt hinweg und ist so gefertigt, etwa durch ein durchgehendes Material, dass die drei Abschnitte z. B. mit Blick von außen auf den Prothesenüberzug nicht oder nicht anhand von umlaufenden Nähten unterscheidbar sind, jedenfalls nicht soweit die drei Abschnitte von der Außenhaut bedeckt sind, da umlaufende Nähte zum Verbinden von jeweils benachbarten Abschnitten miteinander, soweit dies die äußere Schicht betrifft, nicht vorgesehen sind.

In manchen Ausführungsformen sind der erste, zweite und dritte Abschnitt jeweils umlaufend. Sie alle bilden also jeweils eine geschlossene Struktur, die jeweils eine Durchgangsöffnung bildet, in welche die Prothese und/oder der Stumpf des Patienten eingeführt wird, oder durch welche der Stumpf hindurchgeführt wird.

In einigen Ausführungsformen sind der erste, zweite und dritte Abschnitt jeweils umlaufend oder in radialer Richtung luftundurchlässig.

In manchen Ausführungsformen sind der Prothesenüberzug, der erste, der zweite und/oder der dritte Abschnitt und/oder der erste Teilabschnitt jeweils einlagig oder haben jeweils einlagige Abschnitte.

In einigen Ausführungsformen sind der erste und/oder der dritte Abschnitt mehrlagig, indem sie vorzugsweise auf ihrer Außenseite ein erstes Material, z. B. einen elastischen Textilstoff, und unter diesem (auf der Innenseite) eine Schicht anderen Materials aufweisen, z. B. ein Gel oder ein anderes Material, das den gewünschten Tragekomfort gewährleisten kann.

In manchen Ausführungsformen weisen der erste und/oder der dritte Abschnitt, vorzugsweise aber nicht der zweite Abschnitt, ein Material auf, welches die Dehnung des betroffenen Abschnitts in dessen Umfangsrichtung oder radialer Richtung erlaubt oder bestehen aus einem solchen Material.

In einigen Ausführungsformen weist der zweite Abschnitt, und insbesondere sein erster Teilabschnitt oder nur sein erster Teilabschnitt, ein Material auf, welches seine Dehnung in dessen Umfangsrichtung oder radialer Richtung erlaubt oder besteht aus einem solchen Material.

In einigen Ausführungsformen weisen der erste und/oder der dritte Abschnitt, vorzugsweise aber nicht der zweite Abschnitt, ein Material auf, welches die Dehnung des betroffenen Abschnitts in dessen Umfangsrichtung oder radialen Richtung erlaubt oder bestehen einzeln oder jeweils aus einem solchen Material.

Die Dehnbarkeit eines Abschnitts aus einem solchen Material ist in einer Richtung senkrecht zur Umfangsrichtung (also in der Längsrichtung des Prothesenüberzugs) hingegen geringer, etwa im Bereich 0 % bis 70 % der Dehnbarkeit in Umfangsrichtung. Dehnbarkeit kann rein exemplarisch mittels gewellt oder zick-zack verlaufender Fasern in Umfangsrichtung bei z. B. nicht gewellt oder zick-zack verlaufenden Fasern in Längsrichtung erzielt werden.

In einigen Ausführungsformen weisen der erste und/oder der dritte Abschnitt einen anderen Durchmesser und/oder Umfang auf als der zweite Abschnitt, z. B. im getragenen Zustand, im nicht-getragenen Zustand, auf dem Tisch liegend, oder noch in der Verpackung. Dabei ist der Durchmesser und/oder der Umfang des zweiten Abschnitts vorzugsweise größer als der Durchmesser bzw. der Umfang sowohl des ersten als auch des dritten Abschnitts.

Der Prothesenüberzug kann in manchen Ausführungsformen in wenigstens einem (in Längsrichtung) mittleren Abschnitt, wie dem zweiten Abschnitt, breiter als wenigstens ein Abschnitt proximal und als wenigstens ein Abschnitt distal des mittleren Abschnitts sein.

Die Form des Prothesenüberzugs kann in einigen Ausführungsformen in Längsrichtung zunächst breiter (oder genau einmal breiter) und anschließend wieder schmaler (oder genau einmal wieder schmaler) werden.

Der Prothesenüberzug kann in einigen Ausführungsformen in einem mittleren Abschnitt, z. B. dem zweiten Abschnitt, seinen größten Durchmesser und/oder Umfang aufweisen.

Der Prothesenüberzug kann in manchen Ausführungsformen in einem mittleren Abschnitt, z. B. dem zweiten Abschnitt, in welchem er keine Innenauskleidung oder keine Silikonlage aufweist, seinen größten Durchmesser und/oder Umfang aufweisen.

Die Durchmesser können beispielsweise mittels Lineals am flach auf dem Tisch liegenden Prothesenüberzug in einem Zustand, in welchem dieser nicht durch externe Kräfte gedehnt, gestreckt, usw. wird, gemessen werden. Die Umfänge können beispielsweise in einem solchen Zustand mittels Maßband gemessen werden, oder durch Verdoppelung des wie vorstehend bestimmten Durchmessers abgeschätzt werden.

In manchen Ausführungsformen weist der Prothesenüberzug, beispielsweise in einem Nichtgebrauchszustand, keine Krümmung in seiner Längsrichtung auf, insbesondere keine, die fertigungsbedingt ist, etwa durch Verwenden von längeren Strukturen auf einer Vorderseite des Prothesenüberzugs als auch auf einer Rückseite (etwa beim Tragen des Prothesenüberzugs).

In einigen Ausführungsformen weist der Prothesenüberzug Versteifungselemente aus einem bandähnlichen Stoff auf, die außenseitig oder innenseitig auf den Prothesenüberzugstoff oder das Prothesenüberzugmaterial aufgenäht, aufgeklebt oder anderweitig mit ihm verbunden sein können. Eine solche Verbindung kann abschnittsweise oder entlang des gesamten Versteifungselements vorgesehen sein. Vorzugsweise ist zumindest ein mittlerer Abschnitt des Versteifungselements (z. B. in dessen Längsrichtung) nicht mit dem Prothesenüberzugstoff, etwa dem zweiten Abschnitt, verbunden.

Derartige Versteifungselemente können als zweiter Teilabschnitt des zweiten Abschnitts wie hierin beschrieben ausgestaltet sein, und/oder umgekehrt. Was also für einen zweiten Teilabschnitt hierin gesagt ist, kann optional auch für die Versteifungselemente gelten und umgekehrt.

In manchen Ausführungsformen weist die Wandung des zweiten Abschnitts - z. B. in einem vollständig entfalteten Zustand hiervon - keine variable radiale Dicke, vor allem ihrer Wandung, insbesondere über der anterioren oder der ventralen Seite, auf.

In einigen Ausführungsformen weist die Wandung des ersten und/oder des dritten Abschnitts - z. B. in einem vollständig entfalteten Zustand hiervon - unterschiedliche radiale Dicken auf, vor allem ihrer Wandung, wobei vorzugsweise insbesondere die anteriore oder ventrale Seite (beim Tragen des Prothesenüberzugs) dicker als die posteriore oder dorsale Seite sein kann.

In einigen Ausführungsformen weist der Prothesenüberzug keine Verdickung in einem der Abschnitte auf, die von der Verwendung unterschiedlichen Materialien oder Materialstärken herrührt. In anderen ist dies anders, insbesondere was den zweiten Abschnitt betrifft - dieser kann andere oder unterschiedliche Wandstärken aufweisen als der erste und/oder der zweite Abschnitt.

In manchen Ausführungsformen sind der Prothesenüberzug oder einer oder mehrere seiner hierin genannten Abschnitte nicht aus einem gestrickten, einstückigen elastischen Schlauch gefertigt. Dies gilt insbesondere für den zweiten Abschnitt.

In einigen Ausführungsformen weist der Prothesenüberzug keinen Stoffabschnitt auf, der in zwei, insbesondere senkrecht aufeinander stehenden, Richtungen dehnbar ist. Dies gilt insbesondere für den zweiten Abschnitt.

In manchen Ausführungsformen weist der Prothesenüberzug kein Material auf, das eine Bewegung behindert, optional weder auf der Innen- noch auf der Außenseite, insbesondere eine Beugebewegung des Trägers des Prothesenüberzugs.

In einigen Ausführungsformen weist der Prothesenüberzug keine Mehrzahl ringförmiger Vorsprünge auf, insbesondere nicht am oder innerhalb des ersten, des zweiten und/oder des dritten Abschnitts.

In manchen Ausführungsformen weist der Prothesenüberzug zumindest an seiner Außenseite, vorzugsweise zumindest nicht in einem Bereich, welcher dem zweiten Abschnitt zuzuordnen oder Teil hiervon ist - keine längslaufenden Vorsprünge auf, welche sich, jeweils (z. B. längs) um einen Umfang des Prothesenüberzugs verteilt, an diesem erstrecken.

In manchen Ausführungsformen weist die Außenhaut des Prothesenüberzugs keine- insbesondere durch die Materialdicke selbst bedingte - längslaufenden Vorsprünge auf, welche sich, jeweils (z. B. längs) um einen Umfang des Prothesenüberzugs verteilt, an diesem erstrecken. Ungeachtet dessen kann jedoch eine radial innen zur Außenhaut angeordnete Innenauskleidung, welche im ersten und/oder dritten Abschnitt vorliegen kann, auf ihrer Außenseite eine gewellte Oberflächenstruktur aufweisen. Diese kann, z. B. bedingt durch das ausgewählte Fügeverfahren zum Verbinden der Außenhaut mit der darunterliegenden Innenauskleidung, auch die Außenhaut in Wellen oder Falten legen.

In einigen Ausführungsformen ist die Außenhaut eine wenigstens oder genau dreilagige Schicht.

In einigen Ausführungsformen weist der Prothesenüberzug zumindest an seiner Außenseite keine längslaufenden Nähte auf, welche sich in Längsrichtung des Prothesenüberzugs erstrecken. In anderen Ausführungsformen weist der Prothesenüberzug zumindest an seiner Außenseite eine Naht oder einen Stoß oder eine Überlappung auf, welche sich in Längsrichtung des Prothesenüberzugs oder eines oder mehrerer seiner Abschnitte, insbesondere des zweiten Abschnitts, vorzugsweise über den ersten, zweiten sowie dritten Abschnitt, und besonders bevorzugt über die gesamte Länge des Prothesenüberzugs oder zumindest der Außenhaut, erstreckt.

In einigen Ausführungsformen ist die in Längsrichtung verlaufende Naht, Stoß oder Überlappung, sofern vorhanden, auf einer Innenseite und/oder auf einer Außenseite des Prothesenüberzugs in ihrer gesamten Längserstreckung oder über einen überwiegenden Teil ihrer Längserstreckung (d. h. > 50 % ihrer Länge) mittels eines Bands oder mittels Bandmaterials bedeckt.

Das Band kann ein Klebeband sein.

Das Band kann mehrlagig z. B. dreilagig oder mehrlagig sein. Die mehreren Lagen können unterschiedliche Materialien aufweisen und ggf. unterschiedliche Funktionen erfüllen. So kann eine erste Lage eine Klebeschicht sein, welche z. B. mittels Heißluft verflüssigt werden kann, um bis in die Nahtlöcher der optionalen Naht sowie in Poren der von der Naht zusammengehaltenen Schichten, z. B. der der Außenhaut, welche als äußerste Schicht ihrerseits ein Textil aufweisen kann, eindringen und dort aushärten zu können. Eine über dieser Klebeschicht angeordnete zweite Lage kann die Funktion einer Sperrschicht übernehmen und z. B. luftundurchlässig sein. Eine über der zweiten Lage angeordnete dritte Lage kann eine textile Oberfläche aufweisen oder darstellen, wobei unter einer solchen Textilie hierin ein morphologisch bestimmbares, gestaltetes Gefüge aus verspinnbaren, längenbegrenzten Fasern und (oder) gezogenen, endlosen Fasern, die die Verspinnbarkeit als Eigenschaft aufweisen, verstanden werden kann.

Die Außenhaut des Prothesenüberzugs, die äußerste Schicht des ersten Abschnitts, des zweiten Abschnitt und/oder des dritten Abschnitts kann ebenfalls genau oder wenigstens dreilagig sein. Eine erste Lage und/oder eine dritte Lage können dabei jeweils ein Textil (identisch oder verschieden voneinander) sein. Eine zwischen ihnen angeordnete zweite Lage kann dabei eine Membran sein.

Die Membran kann z. B. ein Polyurethan (PU), ein Polysulfon (PSU) oder ein Polyethersulfon (PES) sein oder aufweisen.

Die Membran kann eine Porenweite von 20 $\mu$m bis 70 $\mu$m aufweisen. Die Membran kann ihrerseits aus verschiedenen Lagen bestehen, die sich jeweils in ihrer Porenweite und/oder ihrem Material (z. B. PU, PES, PSU) voneinander unterscheiden können.

In manchen Ausführungsformen weist die Außenhaut eine Membran auf oder besteht hieraus, ist aber nicht dreilagig.

In einigen Ausführungsformen weist der Prothesenüberzug zumindest an seiner Außenseite kein Stabilisierungselement, insbesondere keines aus Kunststoff, insbesondere kein steifes Stabilisierungselement, auf. Hiervon ausgenommen können bandartige, biegsame und/oder flexible Strukturen wie z. B. der zweite Teilabschnitt sein.

In manchen Ausführungsformen weist der Prothesenüberzug keine Strickware und/oder keine Maschenware auf oder besteht nicht aus solcher.

In einigen Ausführungsformen weist der Prothesenüberzug an der inneren Oberfläche keinen Ring oder keine Vielzahl an Ringen auf.

In manchen Ausführungsformen sind keine haftenden oder klebenden Fasern in den Stoff integriert, welche auf der Innenseite des Prothesenüberzugs hervortreten und für zusätzlichen Halt sorgen.

In einigen Ausführungsformen ist der Prothesenüberzug zum Anlegen eines Vakuums geeignet und ausgestaltet. Dafür kann das Material des Prothesenüberzugs, insbesondere des ersten, zweiten und/oder dritten Abschnitts, aus einem luftundurchlässigen Material bestehen, sodass ein angelegtes Vakuum vorzugsweise zumindest über einen bestimmten Zeitraum, beispielsweise über einige Stunden oder Tage, aufrechterhalten werden kann. Ein Ventil weist der Prothesenüberzug in manchen Ausführungsformen nicht auf, insbesondere kein Ventil, das in einer Wandung des ersten, zweiten und/oder dritten Abschnitts liegen würde.

In manchen Ausführungsformen ist wenigstens ein Durchmesser und/oder Umfang (oder der durchschnittliche Durchmesser bzw. Umfang) des ersten Abschnitts und/oder des dritten Abschnitts in einem entspannten Zustand oder einem Nicht-Gebrauchszustand des Prothesenüberzugs kleiner als ein Durchmesser und/oder Umfang (oder der durchschnittliche Durchmesser bzw. Umfang) des zweiten Abschnitts. Der Durchmesser oder der Umfang des zweiten Abschnitts kann optional zwischen 5 % und 20 % größer als der Durchmesser oder Umfang des ersten und/oder des dritten Abschnitts sein. Vorstehende Ausführungen können insbesondere dann gelten, wenn das Material der betrachteten Abschnitte (ggf. nach deren zerstörenden Herauslösen aus dem Prothesenüberzug zum Zwecke des Nachmessens) glattgestrichen oder -gezogen ist.

In einigen Ausführungsformen ist wenigstens eine Erstreckung (oder die durchschnittliche Erstreckung) des zweiten Teilabschnitts in einem spannungsfreien Zustand oder einem Nicht-Gebrauchszustand des Prothesenüberzugs kleiner als eine Erstreckung (oder die durchschnittliche Erstreckung) des ersten Teilabschnitts. Die Erstreckung kann in Längsrichtung des Prothesenüberzugs bestimmt sein und damit der Länge des betrachteten Teilabschnitts entsprechen. Die Erstreckung des ersten Teilabschnitts kann optional zwischen 5 % und 20 % größer als die Erstreckung des zweiten Teilabschnitts sein. Die Erstreckung des ersten Teilabschnitts kann optional zwischen 3 cm und 15 cm größer als die Erstreckung des zweiten Teilabschnitts sein, bevorzugt zwischen 5 cm und 10 cm, ganz besonders bevorzugt zwischen 6 cm und 9 cm. Vorstehende Ausführungen können insbesondere dann gelten, wenn das Material der betrachteten Teilabschnitte (ggf. nach deren zerstörenden Herauslösen aus dem Prothesenüberzug zum Zwecke des Nachmessens) glattgestrichen oder -gezogen ist.

In einigen Ausführungsformen ist wenigstens eine Erstreckung (oder die durchschnittliche Erstreckung) des zweiten Abschnitts, des ersten Teilabschnitts und/oder des zweiten Teilabschnitts in einem spannungsfreien Zustand oder einem Nicht-Gebrauchszustand des Prothesenüberzugs größer als eine Erstreckung (oder die durchschnittliche Erstreckung) des ersten und/oder des dritten Abschnitts. Die Erstreckung kann in Längsrichtung des Prothesenüberzugs bestimmt sein und damit der Länge des betrachteten Teilabschnitts entsprechen. Die Erstreckung des zweiten Abschnitts, des ersten Teilabschnitts und/oder des zweiten Teilabschnitts kann optional zwischen 50 % und 500 % größer als die Erstreckung des ersten und/oder des zweiten Abschnitts sein. Vorstehende Ausführungen können insbesondere dann gelten, wenn das Material der betrachteten Abschnitte und Teilabschnitte (ggf. nach deren zerstörendem Herauslösen aus dem Prothesenüberzug zum Zwecke des Nachmessens) glattgestrichen oder -gezogen sind.

In manchen Ausführungsformen ist die Wandstärke des ersten Abschnitts oder des dritten Abschnitts in einem Bereich stärker, dicker oder größer als in benachbarten Bereichen vor allem des zweiten Abschnitts, z. B. in einem spannungsfreien Zustand des Prothesenüberzugs oder während des üblichen Gebrauchs des Prothesenüberzugs.

In einigen Ausführungsformen enden der zweite Abschnitt, der erste Teilabschnitt und/oder der zweite Teilabschnitt - z. B. in einem unteren oder einem mittleren Bereich, z. B. dessen unterer Hälfte oder unterem Drittel, (in Längsrichtung des Prothesenüberzugs) des dritten Abschnitts - in einem Befestigungsabschnitt oder reichen bis dorthin, in welchem der zweite Abschnitt, der erste Teilabschnitt und/oder der zweite Teilabschnitt (vorzugsweise entlang des gesamten Umfangs des ersten Teilabschnitts oder des zweiten Abschnitts) mit dem dritten Abschnitt verbunden (verklebt, vernäht, verschweißt, usw.) ist. Auf diese Weise steht der dritte Abschnitt - vorzugsweise innenseitig und/oder nur innenseitig - über das untere Ende des zweiten Abschnitts, des ersten Teilabschnitts und/oder des zweiten Teilabschnitts in Richtung zum ersten Abschnitt in Gestalt eines Überstands über.

In manchen Ausführungsformen ist dieser Überstand des dritten Abschnitts ganz oder abschnittsweise nicht mit dem zweiten Abschnitt, dem ersten Teilabschnitt und/oder dem zweiten Teilabschnitt verbunden (verklebt, vernäht, usw.).

Der zweite Abschnitt ist in einem Befestigungsabschnitt mit dem dritten Abschnitt verbunden, wobei der dritte Abschnitt den Befestigungsabschnitt über den Befestigungsabschnitt hinaus den zweiten Abschnitt zumindest abschnittsweise mittels eines Überstands des dritten Abschnitts überragt. In diesem überragenden Abschnitt oder dem Überstand ist das Material des dritten Abschnitts nicht mit dem zweiten Abschnitt verbunden, etwa durch Kleben, Nähen, usw.

In einigen Ausführungsformen ist der zweite Abschnitt in einem, beispielsweise geschlossenen oder ringförmigen, Befestigungsabschnitt mit dem dritten Abschnitt verbunden, wobei der dritte Abschnitt über den Befestigungsabschnitt hinaus in Richtung auf den ersten Abschnitt zumindest abschnittsweise mittels eines Überstands den Befestigungsabschnitt des dritten Abschnitts überragt. In diesem überragenden Abschnitt oder dem Überstand oder Abschnitten hiervon ist das Material des dritten Abschnitts nicht mit dem zweiten Abschnitt verbunden, etwa durch Kleben, Nähen, usw.

In manchen Ausführungsformen erstreckt sich der dritte Abschnitt mittels eines Überstands, mittels welchem er über den Befestigungsabschnitt zwischen drittem und zweitem Abschnitt hinausragt, entlang der Innenseite des zweiten Abschnitts.

Der hierin offenbarte Überstand kann nach Erwerb des Prothesenüberzugs von seinem Träger oder vom Orthopädietechniker auf die individuellen Bedürfnisse des Patienten zugeschnitten werden. Geometrische Besonderheiten der Prothese, welche mit harten Materialien und/oder Kanten einhergehen können, können somit bei Tragen des Prothesenüberzugs durch das weichere und/oder dickere Material des dritten Abschnitts bedeckt sein, während der Überzug dort, wo er keinen solchen Nutzen haben kann, aus Gründen der Gewichtsersparnis und des höheren Tragekomforts weggeschnitten werden kann.

In einigen Ausführungsformen sind die Lamellen in Form von Wellen oder Aufwerfungen vorgesehen.

In manchen Ausführungsformen ist der erste Abschnitt schmaler (in Längsrichtung) und/oder länger (in Umfangsrichtung oder in seiner Abwicklung) als der dritte Abschnitt.

In einigen Ausführungsformen weist der zweite Abschnitt einen größeren Umfang oder eine größere Abwicklung auf als der erste Abschnitt und/oder als der zweite Abschnitt.

In manchen Ausführungsformen hat der zweite Abschnitt einen sich in einer Längsrichtung des zweiten Abschnitts vergrößernden Umfang oder eine in einer Längsrichtung des zweiten Abschnitts größer werdende Abwicklung.

In einigen Ausführungsformen weisen der zweite Abschnitt und/oder seine Teilabschnitte oder deren Materialien eine geringere Dehnbarkeit als der erste Abschnitt und/oder als der dritte Abschnitt oder deren Materialien auf, z. B. in Längsrichtung des Prothesenüberzugs.

Unter Dehnbarkeit versteht man in der Festigkeitslehre, auf welche hier Bezug genommen wird, die Eigenschaft eines Werkstoffes, unter Krafteinwirkung seine Form zu verändern. Die Dehnbarkeit gibt an, wie weit ein Werkstoff verlängert werden kann, ohne dass er bricht oder reißt.

Für die Dehnung gilt:

$$\varepsilon = \frac{\Delta L}{L_0}$$

Unter Elastizität, wie hierin verwendet, ist die Eigenschaft eines Körpers oder Werkstoffes gemeint, unter Krafteinwirkung seine Form zu verändern und bei Wegfall der einwirkenden Kraft in die Ursprungsform zurückzukehren (Beispiel: Sprungfeder).

Der Elastizitätsmodul ist, wie hierin verwendet, z. B. als Steigung des Graphen im Spannungs-Dehnungs-Diagramm bei einachsiger Belastung bei infinitesimaler Verzerrungsänderung bei Spannungsfreiheit definiert. Die meisten Materialien haben einen (zumindest kleinen) linearen Bereich, dieser wird auch als Hookescher Bereich bezeichnet. Es gilt

$$E = \frac{\sigma}{\varepsilon} = \mathrm{const}$$

Dabei bezeichnet $\sigma$ (=Kraft/Fläche) die mechanische Spannung (Normalspannung, nicht Schubspannung) und $\varepsilon = \Delta L / L_0$ die Dehnung. Die Dehnung ist das Verhältnis von Längenänderung $\Delta L = L - L_0$ zur ursprünglichen Länge $L_0$. Die Einheit des Elastizitätsmoduls ist die einer Spannung:

$$E \ \text{in} \ \frac{\mathrm{N}}{\mathrm{mm}^2}, \ \text{in SI-Einheiten:} \ E \ \text{in} \ \frac{\mathrm{N}}{\mathrm{m}^2} \ (\text{Pascal})$$

Der Elastizitätsmodul wird als Materialkonstante bezeichnet, da mit ihm und den Querkontraktionszahlen das Elastizitätsgesetz aufgestellt wird. Der Elastizitätsmodul ist allerdings nicht bezüglich aller physikalischen Größen konstant. Er hängt von verschiedenen Umgebungsbedingungen wie z. B. Temperatur oder Feuchte ab. Daher werden hierin vergleichbare Bedingungen bei der Bestimmung des Elastizitätsmoduls angenommen.

In manchen Ausführungsformen weist der Prothesenüberzug sowohl auf seiner medialen Seite als auch seiner lateralen Seite je wenigstens oder genau einen zweiten Teilabschnitt auf.

In manchen Ausführungsformen weist der Prothesenüberzug eine sich über alle drei Abschnitte erstreckende Schicht, z. B. eine Außenhaut, auf. In einigen Ausführungsform ist diese Schicht im Bereich des zweiten Abschnitts nicht mit einer zweiten Schicht verklebt oder anderweitig verbunden, insbesondere nicht über ihren gesamten Umfang hinweg.

In einigen Ausführungsformen weist der erste Abschnitt an der Innenseite der Außenhaut eine Innenauskleidung auf oder besteht aus der Außenhaut und der Innenauskleidung sowie optional Enden von zweiten Teilabschnitten oder Bändern.

In manchen Ausführungsformen ist die Innenauskleidung zylindrisch (bei entsprechendem Halten des Prothesenüberzugs) oder ein anders geformter Hohlkörper mit einer Durchgangsrichtung in Längsrichtung des Prothesenüberzugs.

Die Innenauskleidung kann umlaufend sein, und sie kann optional eine Länge aufweisen, die größer als ihre Wandstärke oder ihre durchschnittliche Wandstärke ist.

Die Innenauskleidung kann in manchen Ausführungsformen z. B. aus Silikon sein oder Silikon aufweisen.

Die Wandstärke der Innenauskleidung liegt in manchen Ausführungsformen, in einem unbelasteten Zustand des Prothesenüberzugs, z. B. in einem Bereich von 3 mm bis 5 mm, vorzugsweise 4 mm.

Die Außenhaut, oder ihr Material, kann in einigen Ausführungsformen luftdicht sein. Sie kann, optional, ein Membrangewebe sein oder aufweisen. Sie kann optional zwei-, drei- oder mehrlagig sein.

Insbesondere können die Außenhaut und die Innenauskleidung aus unterschiedlichen Materialien sein oder unterschiedliche Materialien aufweisen.

Außenhaut und Innenauskleidung können im Bereich des ersten Abschnitts und/oder des dritten Abschnitts miteinander verklebt oder auf andere Weise miteinander verbunden sein. Die Verklebung oder andere Verbindung kann sich über die gesamte Länge des ersten oder dritten Abschnitts oder der dort vorliegenden Innenauskleidung oder nur einen Teil hiervon erstrecken (in Längsrichtung des Prothesenüberzugs).

In diese Verbindung oder Verklebung hinein kann in einigen Ausführungsformen ein Endabschnitt des Teilabschnitts, welcher z. B. ein laterales Band wie hierin beschrieben und/oder ein mediales Band haben oder sein kann, eingearbeitet sein, z. B. durch gemeinsames Verbinden oder Verkleben. Der erste und/oder der dritte Abschnitt kann somit abschnittsweise zu einem genau dreilagigen oder wenigstens dreilagigen Klebeabschnitt werden, an anderen Stellen hingegen eine Lage weniger aufweisen.

Die Außenhaut weist in manchen Ausführungsformen keine umlaufende Naht auf (also keine in Umfangsrichtung verlaufende Naht), womit Fertigungs- und Haltbarkeitsvorteile verbunden sein können.

Die Außenhaut ist in einigen Ausführungsformen - zumindest in Längsrichtung - durchgehend ausgestaltet. Sie kann z. B. aus einem Textil, einer Membran, Folie, usw. und/oder Kombinationen hiervon gefertigt sein.

Anders als der erste Abschnitt und/oder der dritte Abschnitt weist der zweite Abschnitt in einigen Ausführungsformen vorzugsweise keine Innenauskleidung auf oder eine Innenauskleidung, welche sich von der Innenauskleidung des ersten und/oder dritten Abschnitts in Material und/oder Dicke unterscheidet.

Die Innenauskleidung des ersten Abschnitts und des drittes Abschnitts entsprechen sich in einigen Ausführungsformen in Material und/oder Dicke.

In einigen Ausführungsformen weist die Innenauskleidung des ersten Abschnitts und/oder des dritten Abschnitts - in Umfangsrichtung - eine längere Außenseite als Innenseite auf, was nicht allein darauf zurückzuführen ist, dass auf der Außenseite der Innenauskleidung ein größerer Durchmesser als auf der Innenseite hiervon vorliegt. Die längere Außenseite erklärt sich vielmehr dadurch, dass diese z. B. in Wellen, Falten, usw. geformt ist, wohingegen die Innenseite glatt oder weniger wellig, gefaltet usw. gefertigt ist.

Die wellige, kurvige, gefaltete, aufgeworfene usw. Außenseite kann mit der Außenhaut derart verklebt sein, dass sich diese in jenen Bereichen, in welchen sie mit der Innenauskleidung z. B. verklebt ist, ebenfalls in Wellen, Falten usw. legt.

Der maximale Umfang des zweiten Abschnitts beträgt in einigen Ausführungsformen 10 %, 20 %, 30 %, 50 %, 100 %, 150 % oder mehr des Umfangs im Bereich des ersten Abschnitts und/oder im Bereich des dritten Abschnitts.

In manchen Ausführungsformen ist nur ein proximaler oder distaler Teil der Innenauskleidung mit der Außenhaut direkt oder indirekt verbunden oder verklebt, der distale bzw. proximale Teil hingegen nicht.

In manchen Ausführungsformen ist der Umfang am proximalen Ende der Innenauskleidung des ersten Abschnitts weiter oder länger als der Umfang am gegenüberliegenden, distalen Ende dieser Innenauskleidung. Diese Innenauskleidung kann damit eine leicht konische Form und/oder eine Neigung ihrer Außenfläche zu einer Mittellinie (z. B. in einem Querschnitt) haben. Die Neigung kann bis zu 5° oder mehr, vorzugsweise zwischen 0,5° und 3° betragen. Letzteres kann Fertigungsvorteile aufweisen. Die Neigung kann optional nur auf den proximalen Abschnitt dieser Innenauskleidung zutreffen.

Die Länge der Innenauskleidung des ersten Abschnitts kann z. B. zwischen 90 cm und 120 cm betragen, der Umfang kann z. B. zwischen 250 cm und 450 cm betragen und/oder der Durchmesser kann z. B. zwischen 80 cm und 145 cm liegen.

In manchen Ausführungsformen ist der Umfang am proximalen Ende der Innenauskleidung des dritten Abschnitts weiter oder länger als der Umfang am gegenüberliegenden, distalen Ende dieser Innenauskleidung. Diese Innenauskleidung kann damit eine leicht konische Form und/oder eine Neigung ihrer Außenfläche zu einer Mittellinie (in einem Querschnitt) haben. Die Neigung kann bis zu 5° oder mehr, vorzugsweise zwischen 0,5° und 3° betragen. Letzteres kann Fertigungsvorteile aufweisen.

Die Länge der Innenauskleidung des dritten Abschnitts kann z. B. zwischen 140 cm und 250 cm betragen, der Umfang kann z. B. zwischen 210 cm und 370 cm betragen und/oder der Durchmesser kann z. B. zwischen 67 cm und 118 cm liegen.

In manchen Ausführungsformen ist der Umfang am proximalen Ende der Außenhaut (z. B. vor ihrer optionalen Verbindung mit der oder den Innenauskleidungen usw.) weiter oder länger als der Umfang am gegenüberliegenden, distalen Ende der Außenhaut.

In einigen Ausführungsformen ist die Wandstärke oder durchschnittliche Wandstärke des zweiten Abschnitts geringer, zumindest abschnittsweise oder durchgehend, als die Wandstärke oder durchschnittliche Wandstärke des ersten und/oder des dritten Abschnitts.

In manchen Ausführungsformen ist die Materialstärke oder durchschnittliche Materialstärke der Außenhaut geringer, zumindest abschnittsweise oder durchgehend, als die Materialstärke oder durchschnittliche Materialstärke der Innenauskleidung des ersten und/oder dritten Abschnitts.

In einigen Ausführungsformen ist die Materialstärke oder durchschnittliche Materialstärke der Außenhaut um wenigstens 1,5 mm geringer, zumindest abschnittsweise oder durchgehend, als die Materialstärke oder durchschnittliche Materialstärke der Innenauskleidung des ersten und/oder dritten Abschnitts.

In manchen Ausführungsformen ist die Außenhaut der erste Teilabschnitt. Was hierin für die Außenhaut ausgeführt ist, kann ungeschmälert für den ersten Teilabschnitt gelten, und/oder umgekehrt.

Von der Erfindung umfasst ist auch, dass nicht, oder nicht nur, gilt, dass das Material oder dass ein Material des zweiten Abschnitts einen größeren Elastizitätsmodul aufweist als eines oder mehrere der Materialien des ersten Abschnitts und/oder des dritten Abschnitts, sondern dass alternativ oder ergänzend der zweite Abschnitt einen größeren Elastizitätsmodul in genau einer oder wenigstens einer Richtung aufweist als der erste Abschnitt und/oder der dritte Abschnitt. Alle hierin genannten Merkmale und Ausführungsformen lassen sich erfindungsgemäß auch auf diese Ausgestaltung lesen bzw. mit dieser kombinieren.

Manche oder alle erfindungsgemäßen Ausführungsformen können einen, mehrere oder alle der oben und/oder im Folgenden genannten Vorteile aufweisen.

Von Vorteil kann sein, dass der Prothesenüberzug, wenn er ein Gelenk überbrückend beim Patienten angelegt ist, aufgrund einer Materialreserve, die der Prothesenüberzug dem Träger z. B. auf seiner anterioren oder ventralen Seite mittels des zweiten Abschnitts bietet, einen hohen Tragekomfort bieten kann. So bedarf es je nach Ausführung des zweiten Abschnitts keiner Kräfte, oder geringerer Kräfte als bei bekannten Prothesenüberzügen, beim Beugen des mittels des Prothesenüberzugs bedeckten oder überbrückten Gelenks, etwa des künstlichen Knies beim Gehen.

Ein Reiben auf der Haut oder auf einer unter dem Prothesenüberzug liegenden Schicht wie z. B. einem Liner, bedingt durch ein Dehnen des Prothesenüberzugs auf der Streckseite des Gelenks gefolgt durch ein Kontrahieren des Prothesenüberzugs über oder auf der Prothese oder der Haut des Patienten, kann in einigen Ausführungsformen ebenfalls vorteilhaft verhindert oder verringert werden, da der auf der Streckseite des Gelenks liegende zweite Abschnitt nicht elastisch oder nur in vergleichsweise geringem Maße elastisch ist und daher keinen Druck auf die Haut ausübt und/oder sich nicht auf die Haut auflegt.

Aufgrund der besonderen Auswahl der hierin beschriebenen E-Module des Prothesenüberzugs kann ferner sichergestellt werden, dass der Prothesenüberzug zumindest im Bereich des nicht elastischen, weniger elastischen oder nur in vergleichsweise geringem Maße elastischen Abschnitts keinen Druck auf die darunterliegenden Strukturen oder Gewebe ausübt, unabhängig von einer Beugung des Gelenks.

Der zweite Teilabschnitt kann - sofern vorgesehen - vorteilhaft verhindern, dass sich der zweite Abschnitt bereits beim Anziehen oder Anlegen des Prothesenüberzugs maximal längt oder streckt und damit die Materialreserve, die für den Tragekomfort mitverantwortlich ist, ungewollt minimiert oder aufhebt.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:

Fig. 1     zeigt einen erfindungsgemäßen Prothesenüberzug in einer ersten Ausführungsform;

Fig. 2     zeigt einen erfindungsgemäßen Prothesenüberzug in einer zweiten Ausführungsform;

Fig. 2a     zeigt den unteren Abschnitt des erfindungsgemäßen Prothesenüberzugs der Fig. 2 in einem Teilschnitt;

Fig. 3     zeigt einen erfindungsgemäßen Prothesenüberzug in einer dritten Ausführungsform;

Fig. 3a     zeigt den erfindungsgemäßen Prothesenüberzug der dritten Ausführungsform;

Fig. 3b     zeigt einen erfindungsgemäßen Prothesenüberzug in einer vierten Ausführungsform;

Fig. 3c     zeigt einen erfindungsgemäßen Prothesenüberzug in einer fünften Ausführungsform;

Fig. 3d     zeigt einen erfindungsgemäßen Prothesenüberzug in einer sechsten Ausführungsform;

Fig. 3e     zeigt einen erfindungsgemäßen Prothesenüberzug in einer siebten Ausführungsform;

Fig. 4     zeigt einen erfindungsgemäßen Prothesenüberzug in einer achten Ausführungsform;

Fig. 5     zeigt einen erfindungsgemäßen Prothesenüberzug in einer neunten Ausführungsform; und

Fig. 5a     zeigt einen Schnitt durch den erfindungsgemäßen Prothesenüberzug der Fig. 5 entlang einer Linie B-B der Fig. 5.

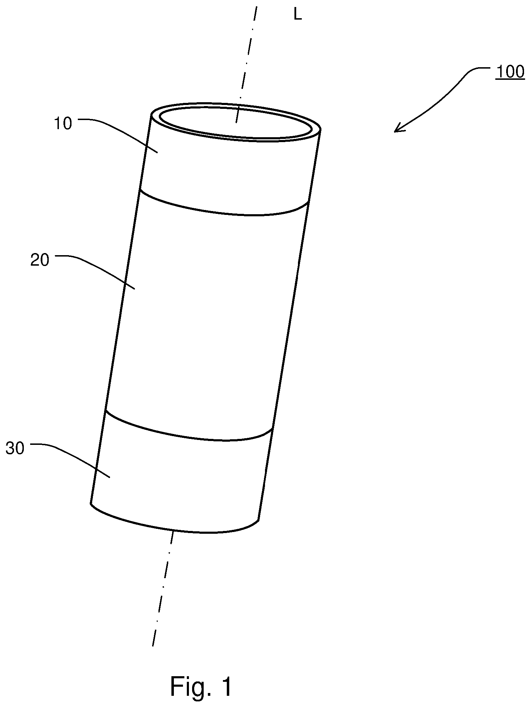

**Fig. 1** zeigt einen erfindungsgemäßen Prothesenüberzug 100 in einer ersten Ausführungsform von der Seite in leichter Perspektive von oben.

Der Prothesenüberzug 100 weist einen ersten Abschnitt 10, einen zweiten Abschnitt 20 und einen dritten Abschnitt 30 auf, oder besteht alternativ hieraus. Seine Längsrichtung oder Längsachse ist mit L bezeichnet. Er verläuft in axialer Richtung. Senkrecht zur Längsrichtung L verläuft die Querrichtung oder die radiale Richtung.

Der zweite Abschnitt 20 liegt dabei zwischen dem ersten Abschnitt 10 und dem dritten Abschnitt 30. Im Beispiel der Figuren berühren sich der erste Abschnitt 10 und der dritte Abschnitt 30 nicht. Sie sind wie in Fig. 1 optional in wenigstens einer radialen Schicht, nicht in Kontakt miteinander.

Der zweite Abschnitt 20 weist einen größeren E-Modul als der erste Abschnitt 10 und/oder der dritte Abschnitt 30 auf.

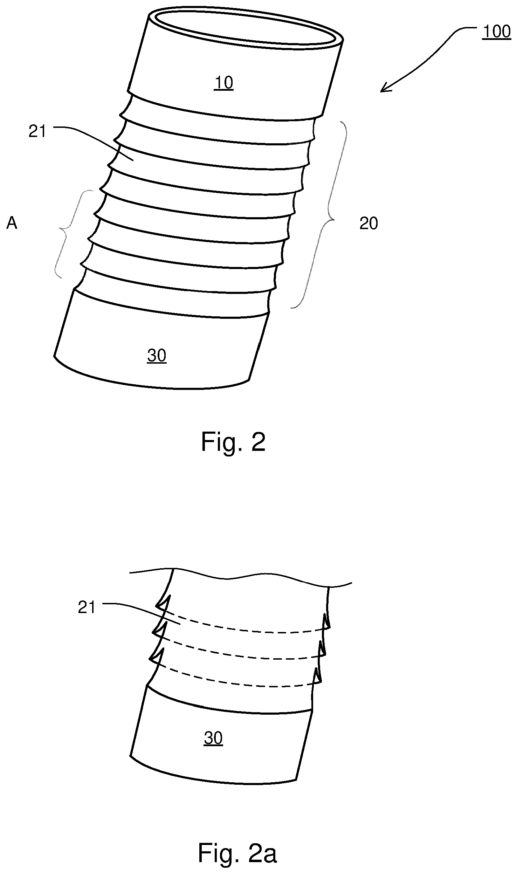

**Fig. 2** zeigt einen erfindungsgemäßen Prothesenüberzug 100 in einer zweiten Ausführungsform.

Dabei ist der zweite Abschnitt 20 in einem ersten Teilabschnitt 21 hiervon längs seines gesamten Umfangs lamellenartig gefaltet. In Fig. 2 sind beispielhaft acht Lamellen zu sehen, jede andere Anzahl ist ebenfalls von der vorliegenden Erfindung umfasst.

Die Lamellen dienen als Beispiel eines Materialreservoirs, insbesondere in Längsrichtung des zweiten Abschnitts 20 (siehe die Linie L in Fig. 1). Wird der Prothesenüberzug 100 über ein Gelenk, etwa über das künstliche Kniegelenk angelegt, und zeigt die Patella oder künstliche Patella des Trägers des Prothesenüberzugs 100 dabei bezogen auf Fig. 2 nach links (mit der Kniekehle auf der rechten Seite des Prothesenüberzugs 100 bezogen auf Fig. 2), so kann sich der zweite Abschnitt 20 mit einem größeren E-Modul gegenüber den Abschnitten 10 und/oder 30 z. B. über dem Knie durch Entfalten verlängern.

Der Patient spürt hierbei keinen Druck auf der Haut, der von der Spannung eines elastischen Materials im zweiten Abschnitt 20 herrühren würde. Der zweite Abschnitt 20 (verglichen mit einem kleineren E-Modul des ersten Abschnitts 10 und/oder des dritten Abschnitts 30) lässt einen solchen Druck nicht entstehen. Statt Druck aufzubauen, verlängert er sich einfach in Längsrichtung aufgrund der hierfür vorgesehenen Lamellenstruktur.

**Fig. 2a** zeigt den unteren Abschnitt des erfindungsgemäßen Prothesenüberzugs 100 der Fig. 2 in einem Teilschnitt A. Der in Fig. 2a gezeigte Abschnitt ist in Fig. 2 mit dem Bezugszeichen A bezeichnet.

Zu erkennen ist, dass das Material des ersten Teilabschnitts 21 des zweiten Abschnitts 20, das in Lamellen gefaltet ist, aufgrund einer Doppelung aufeinander liegt. Das Material ist in dem in Fig. 2 und Fig. 2a gezeigten Zustand des Prothesenüberzugs 100 zwar noch immer in sich gedoppelt oder weist noch immer Doppelungen auf, deren Haupterstreckung liegt jedoch (mehr oder weniger) parallel zur Längsachse des Prothesenüberzugs 100, in der gezeigten Ausführungsform jedenfalls aber nicht senkrecht zu dieser.

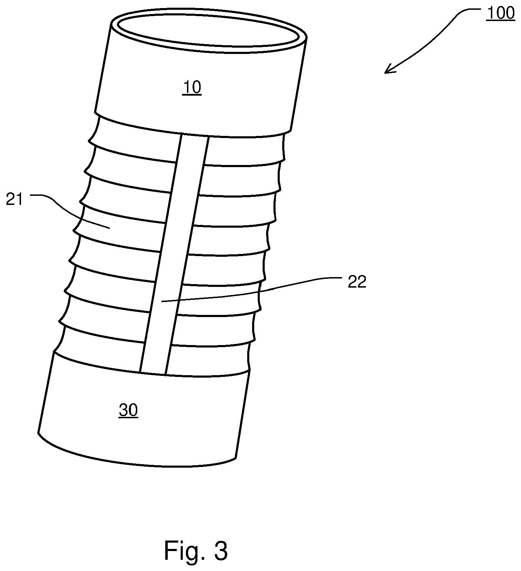

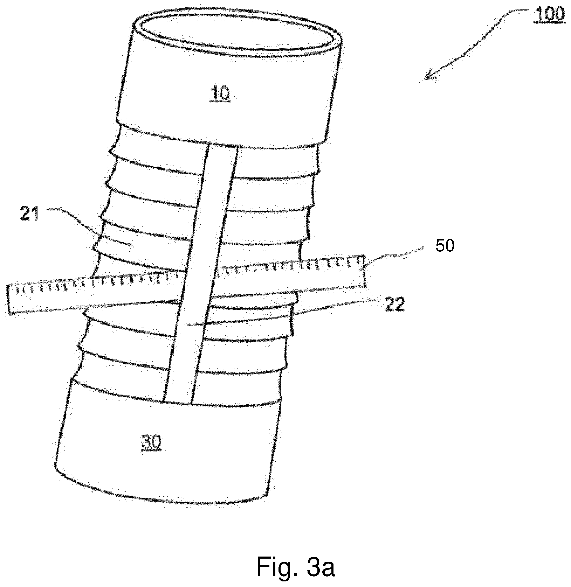

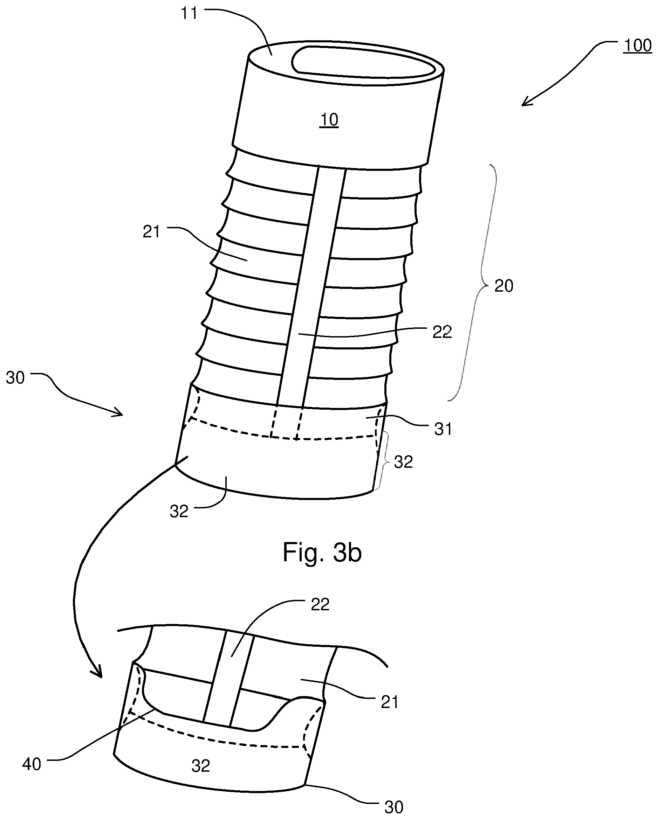

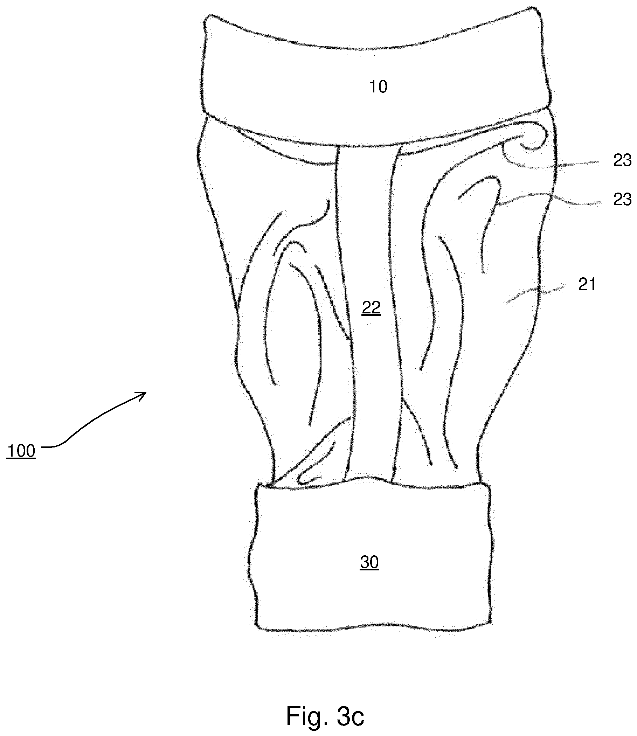

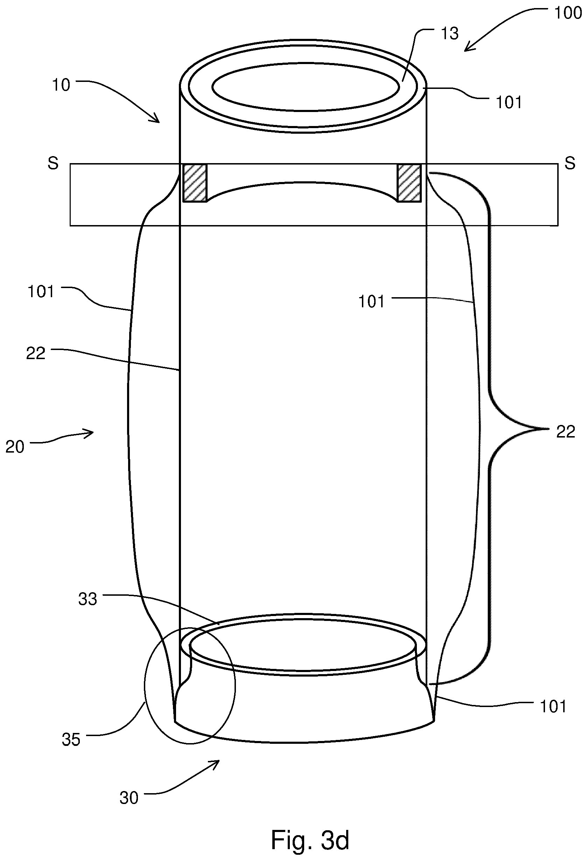

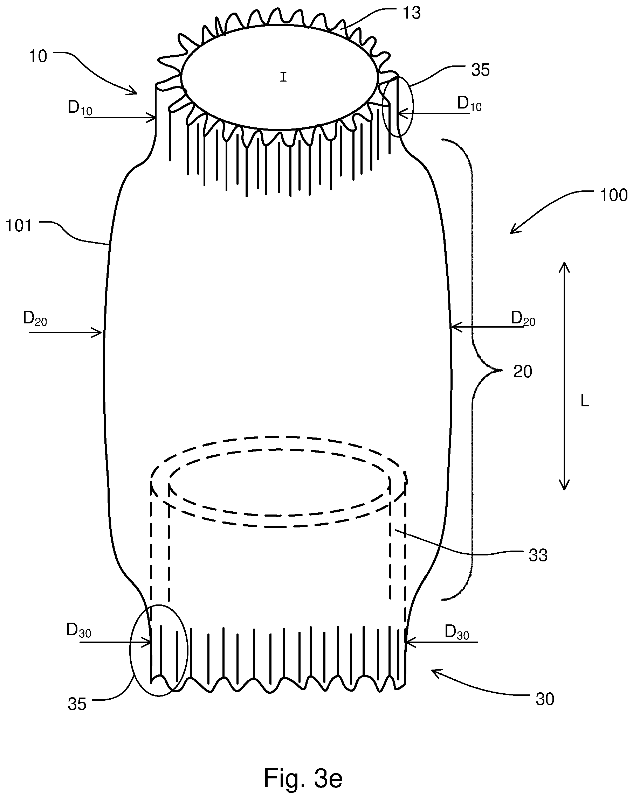

**Fig. 3** zeigt einen erfindungsgemäßen Prothesenüberzug 100 in einer dritten Ausführungsform.

Der Prothesenüberzug 100 unterscheidet sich von jener der Fig. 2 vorzugsweise nur durch einen zweiten Teilabschnitt 22.

Der zweite Teilabschnitt 22 verbindet den ersten Abschnitt 10 - direkt oder indirekt - mit dem dritten Abschnitt 30. Er kann sicherstellen, dass der Abstand zwischen dem ersten Abschnitt 10 und dem dritten Abschnitt 30 (in Längsrichtung L) einen Höchstabstand oder einen gewünschten Maximalabstand nicht überschreitet. Er kann ferner sicherstellen, dass das Materialreservoir des zweiten Abschnitts 20 sich nicht bereits beim Anziehen oder Anlegen des Prothesenüberzugs 100 entfaltet und dem Patienten bei späteren Beugebewegungen nicht mehr zur Verfügung steht.

Anders als der erste Teilabschnitt 21, dessen Länge oder sichtbare oder ausgefaltete Länge durch Entfalten vergrößert werden kann, weist der zweite Teilabschnitt 22 keine Materialreserve auf. Seine Länge ist unveränderbar, jedenfalls im Rahmen seiner Elastizität oder Nicht-Elastizität.

Dabei kann der zweite Teilabschnitt 22 verglichen mit der Elastizität des ersten Abschnitts 10 und/oder des dritten Abschnitts 30 weniger elastisch sein. Er kann optional nicht-elastisch sein. Dasselbe kann gelten für den zweiten Abschnitt 20 in Längsrichtung, der aufgrund des zweiten Teilabschnitts 22 weniger elastisch ist als der erste und/oder der dritte Abschnitt 10, 30.

In Fig. 3 ist ein zweiter Teilabschnitt 22 zu sehen. Jedoch können zwei oder mehr solcher zweiten Teilabschnitte 22 über den Umfang des Prothesenüberzugs 100 verteilt vorgesehen sein, etwa ein zweiter Teilabschnitt 22 auf der medialen Seite des Prothesenüberzugs 100 und ein weiterer Teilabschnitt 22 auf der lateralen Seite des Prothesenüberzugs 100. Weitere zweite Teilabschnitte 22 können vorgesehen sein, es kann auch vorgesehen sein, solche weiteren Teilabschnitte 22 nicht zu haben.

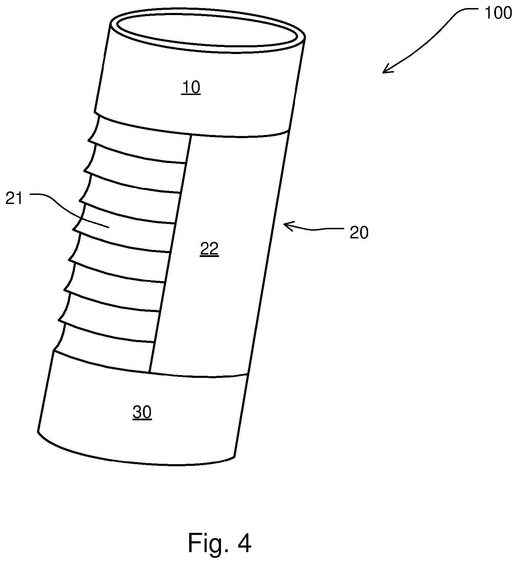

Auch die Breite des zweiten Teilabschnitts 22 kann variieren. In Fig. 3 ist der zweite Teilabschnitt 22 vergleichsweise schmal und/oder als Band gezeigt. Er kann alternativ aber auch breiter als in Fig. 3 gezeigt sein. So kann er z. B. bis zu 100°, 120°, 140°, 160° oder 180° des Umfangs des Prothesenüberzugs 100 oder mehr ausmachen. Letztere Ausführungsform ist in Fig. 4 gezeigt.

Vorzugsweise liegt der zweite Teilabschnitt 22 in wenigstens einem Abschnitt dem ersten Teilabschnitt 21 lose auf (auf der Außenseite oder der Innenseite des ersten Teilabschnitts 21). Er ist in diesem Abschnitt, in dem er lose aufliegt, mit dem darunterliegenden ersten Teilabschnitt 21 also z. B. nicht verklebt oder vernäht oder anders verbunden.

Der lose aufliegende Abschnitt kann 30 %, 40 %, 50 %, 60 %, 70 %, 80 % oder mehr der Länge oder der sichtbaren Länge des zweiten Teilabschnitts 22 betragen.

Bedingt durch das lose Aufliegen des vorgenannten Abschnitts des zweiten Teilabschnitts 22 auf dem ersten Teilabschnitt 21 kann z. B. ein Finger oder eine flache Hand zwischen dem zweiten Teilabschnitt 22 und dem erstem Teilabschnitt 21 hindurchgeschoben werden. Diese Ausgestaltung erlaubt es dem unter dem zweiten Teilabschnitt 22 liegenden ersten Teilabschnitt 21 sich zumindest im Bereich des lose aufliegenden Abschnitts frei zu entfalten, sollte dies beim Tragen des Prothesenüberzugs 100 erforderlich sein. Die durch den lose aufliegenden Abschnitt mögliche Relativbewegung zwischen zweitem Teilabschnitt 22 und erstem Teilabschnitt 21 trägt hierzu vorteilhaft bei.

Obgleich vorstehend davon die Rede ist, dass der zweite Teilabschnitt 22 dem ersten Teilabschnitt 21 aufliegt, soll dies die Erfindung (wie in Fig. 3 gezeigt oder in jeder anderen Ausführungsform oder Merkmalskombination offenbart) nicht beschränken. Tatsächlich kann der zweite Teilabschnitt 22 alternativ oder ergänzend auch unter dem ersten Teilabschnitt 21 liegen, oder zwischen zwei Schichten des ersten Teilabschnitts 21. Dabei könnte der lose aufliegende Abschnitt des zweiten Teilabschnitts 22 unter dem ersten Teilabschnitt 21 oder zwischen zwei Schichten des ersten Teilabschnitts 21 liegen und weder mit der oberen noch der untern Schicht verbunden sein. Somit ist eine Relativbewegung zwischen dem zweiten Teilabschnitt 22 und beiden Schichten des ersten Teilabschnitts 21 möglich.

**Fig. 3a** zeigt den erfindungsgemäßen Prothesenüberzug 100 der dritten Ausführungsform.

In Fig. 3a ist gegenüber der Darstellung des Prothesenüberzug 100 in Fig. 3 der lose aufliegende Abschnitt betont, indem ein Zeichenlineal 50 gezeigt ist, das zwischen dem ersten Teilabschnitt 21 und dem zweiten Teilabschnitt 22 durchgeführt ist, wobei der Teilabschnitt 21 unter dem zweiten Teilabschnitt 22 liegend gezeigt ist, was in beliebigen anderen Ausführungsformen umgekehrt der Fall ist. Dort liegen der erste Teilabschnitt 21, gleich wie dieser ausgestaltet ist, über dem zweiten Teilabschnitt 22 und damit optional auch über dem lose aufliegenden Abschnitt.

Das Zeichenlineal 50 ist unter dem zweiten Teilabschnitt 22 und über dem ersten Teilabschnitt 21 durchgeschoben. Der lose aufliegende Abschnitt, also jener Abschnitt, in welchem der ersten Teilabschnitt 21 nicht mit dem zweiten Teilabschnitt 22 stoffschlüssig (Kleben, Textilschweißen oder dergleichen) oder kraft- oder formschlüssig (Nähen, Kletten, oder dergleichen) oder auf andere Weise verbunden ist, erlaubt ein solches Durchschieben.

Es hat sich bei nicht-öffentlichen Experimenten des Erfinders gezeigt, dass ein loser Abschnitt den Tragekomfort des Patienten oder Nutzers des Prothesenüberzugs maßgeblich verbessern kann.

**Fig. 3b** zeigt einen erfindungsgemäßen Prothesenüberzug 100 in einer vierten Ausführungsform.

Zu erkennen ist, dass die Wandstärke des ersten Abschnitts 10 in einem Bereich 11, der z. B. beim Tragen des Prothesenüberzugs 100 nach vorne (ventral) zeigen kann, optional stärker sein kann als in benachbarten Bereichen.

In Fig. 3b ist ferner, und unabhängig von der Verstärkung der Wand im ersten Abschnitt 10 zu betrachten, eine optionale Weise einer Befestigung des zweiten Abschnitts 20, des ersten Teilabschnitts 21 und/oder des zweiten Teilabschnitts 22 am dritten Abschnitt 30 gezeigt.

Wie Fig. 3b zu entnehmen ist, enden der zweite Abschnitt 20, der erste Teilabschnitt 21 und/oder der zweite Teilabschnitt 22 - z. B. in einem unteren oder einem mittleren Bereich (in Längsrichtung des Prothesenüberzugs 100) des dritten Abschnitts 30 - in einem Verbindungs- oder Befestigungsabschnitt 32, in welchem der zweite Abschnitt 20, der erste Teilabschnitt 21 und/oder der zweite Teilabschnitt 22 (vorzugsweise entlang des gesamten Umfangs des ersten Teilabschnitts 21 oder des zweiten Abschnitts 20) mit dem dritten Abschnitt 30 verbunden (verklebt, vernäht, verschweißt, usw.) sind. Auf diese Weise steht der dritte Abschnitt 30 über das untere Ende des zweiten Abschnitts 20, des ersten Teilabschnitts 21 und/oder des zweiten Teilabschnitts 22 über den Befestigungsabschnitt 32 in Richtung zum ersten Abschnitt 10 über. Der so überstehende Abschnitt wird hier als Überstand 31 bezeichnet.

Ist dieser Überstand 31 des dritten Abschnitts 30 ganz oder abschnittsweise nicht mit dem zweitem Abschnitt 20, dem ersten Teilabschnitt 21 und/oder dem zweiten Teilabschnitt 22 verbunden (verklebt, vernäht, usw.), so kann er vom Benutzer des Prothesenüberzugs 100 auf einfache Weise für dessen Bedürfnisse zurechtgeschnitten werden. So kann der Überstand 31 entlang einer vom Benutzer festzulegenden Schnittlinie 40 beispielsweise auf den oberen Rand einer Prothese abgestimmt zugeschnitten werden.

In Fig. 3b ist dies in einer ausschnittsweisen Darstellung des Prothesenüberzugs 100 gezeigt.

Es spielt dabei keine Rolle, ob der Befestigungsabschnitt 32, in welchem der zweite Abschnitt 20 bzw. seine Teilabschnitte 21, 22 mit dem dritten Abschnitt 30 verbunden sind, bis an ein unteres Ende des dritten Abschnitts 30 reicht oder nur ein Streifen oder anders geformter Bereich ist, über den hinaus der dritte Abschnitt 30 in Richtung weg vom ersten Abschnitt 10 wiederum nicht mit dem zweiten Abschnitt 20 verbunden (verklebt, verschweißt, usw.) ist, z. B. bezogen auf die Fig. 3b nach unten. Jede dieser Ausgestaltungen sind von der vorliegenden Erfindung umfasst.

Von der Erfindung ist ferner umfasst, dass der Überstand 31 des dritten Abschnitts 30 dem zweitem Abschnitt 20 außen oder innen anliegt, was für jede beliebige Ausführungsform gelten kann.

**Fig. 3c** zeigt einen erfindungsgemäßen Prothesenüberzug 100 in einer fünften Ausführungsform, in welche die Lamellen vorangehender Ausführungsformen die Form von Wellen oder Aufwerfungen haben. Exemplarisch sind zwei Bereiche, in welche der zweite Abschnitt 20 gedoppelt vorliegt, mit dem Bezugszeichen 23 bezeichnet.

In Fig. 3c ist ferner gezeigt, dass der erste Abschnitt 10 in jeder beliebigen Ausführungsform schmaler (in Längsrichtung) und/oder länger (in Umfangsrichtung oder in seiner Abwicklung) sein kann als der dritte Abschnitt 30.

In Fig. 3c ist weiter gezeigt, dass der zweite Abschnitt 20 einen größeren Umfang oder eine größere Abwicklung haben kann als der erste Abschnitt 10 und/oder der dritte Abschnitt 30.

Fig. 3c zeigt weiter, dass der zweite Abschnitt 20 einen sich in einer Längsrichtung des zweiten Abschnitts 20 vergrößernden Umfang oder eine in einer Längsrichtung des zweiten Abschnitts 20 größer werdende Abwicklung haben kann.

Der auch in Fig. 3c außenseitig gezeigte zweite Teilabschnitt 22 liegt in anderen Ausführungsformen auf der Innenseite des zweiten Abschnitts 20.

**Fig. 3d** zeigt einen erfindungsgemäßen Prothesenüberzug 100 in einer sechsten Ausführungsform. In einem Bereich S ist er geschnitten dargestellt, unterhalb dieses Bereichs schematisch vereinfacht und teils geschnitten.

In der sechsten Ausführungsform weist der Prothesenüberzug 100 eine sich über alle drei Abschnitte 10, 20 und 30 erstreckenden Schicht, welche den Prothesenüberzug 100 hier an dessen Außenseite bedeckt, weshalb sie hierin - nicht beschränkend - als Außenhaut 101 bezeichnet ist. In anderen als der hier gezeigten Ausführungsform kann diese sich in einer Längsrichtung des Prothesenüberzugs 100 über alle Abschnitte 10, 20 und 30 jeweils ganz oder zumindest abschnittsweise erstreckende Schicht anstatt auf der Außenseite an einer Innerseite oder in einem mittleren Bereich der Wandung des Prothesenüberzugs 100 (bezogen auf dessen Dicke oder Materialstärke) befinden.

Zwar endet die Außenhaut 100 als Beispiel der vorstehend genannten Schicht in der in Fig. 3d gezeigten, exemplarischen Ausführungsform am proximalen sowie am distalen Ende des Prothesenüberzugs 100. Von der Erfindung ist jedoch auch umfasst, dass die Außenhaut 101 sich zwar über alle drei Abschnitte 10, 20 und 30 erstreckt bzw. in diese hineinerstreckt, dabei jedoch - z. B. wenige Millimeter oder Zentimeter - vor dem proximalen und/oder vor dem distalen Ende des Prothesenüberzugs 100 (in dessen Längsrichtung) endet.

Der erste Abschnitt 10 weist an der Innenseite der vorgenannten Außenhaut 101 eine Innenauskleidung 13 auf oder besteht aus der Außenhaut 101 und der Innenauskleidung 13. Die Lage von Außenhaut 101 und Innenauskleidung 13 zueinander, nach welcher die Außenhaut 101 ihrem Namen entsprechend außen auf der Innenauskleidung 13 aufliegt, ist wie vorstehend erwähnt jedoch nur exemplarisch. Alternativ könnte das hier als Innenauskleidung 13 bezeichnete Material oder Element auf der Außenseite der Außenhaut 101 liegen und/oder radial zu dieser. Letztere wäre dann eine Innenhaut, und die Innenauskleidung 13 sprachlich eher eine Umkleidung.

Die Innenauskleidung 13 kann zylindrisch (durch entsprechendes Halten des Prothesenüberzugs) sein oder auch ein anders geformter Hohlkörper mit einer Durchgangsrichtung in Längsrichtung des Prothesenüberzugs.

Die Innenauskleidung 13 kann umlaufend sein, und sie kann optional eine Länge aufweisen, die größer als ihre Wandstärke oder ihre durchschnittliche Wandstärke ist.

Die Innenauskleidung 13 kann z. B. Silikon sein oder Silikon aufweisen, oder ein anderes Material sein oder aufweisen. Die Innenauskleidung 13 kann ausgestaltet sein, um sich vorzugsweise vorteilhaft geschmeidig und vorzugsweise luftdicht an z. B. der Haut des Patienten anzulegen.

Die Wandstärke der Innenauskleidung 13 kann in einem unbelasteten Zustand des Prothesenüberzugs z. B. in einem Bereich von 3 mm bis 5 mm, vorzugsweise 4 mm, liegen.

Die Außenhaut 101 oder ihr Material kann luftdicht sein. Sie kann, optional, ein Membrangewebe sein. Sie kann optional zwei-, drei- oder mehrlagig sein.

Insbesondere können die Außenhaut 101 und die Innenauskleidung 13 aus unterschiedlichen Materialien sein.

Außenhaut 101 und Innenauskleidung 13 können im Bereich des ersten Abschnitts 10 miteinander verklebt oder auf andere Weise miteinander verbunden sein. Die Verklebung oder andere Verbindung kann sich über die gesamte Länge des ersten Abschnitts 10 oder nur einen Teil hiervon erstrecken (in Längsrichtung des Prothesenüberzugs).

In diese Verbindung oder Verklebung hinein kann ein Endabschnitt des Teilabschnitts 22, welcher z. B. ein laterales Band wie hierin beschrieben (rechts in Fig. 3d) und ein mediales, solches Band (links in Fig. 3d) haben oder sein kann, eingearbeitet sein, z. B. durch gemeinsames Verbinden oder Verkleben. Mehr oder weniger als zwei solcher Bänder können ebenfalls vorgesehen sein. Ihre Anordnung ist nicht auf medial oder lateral oder auf 180° versetzt zueinander, bezogen auf den Umfang des Prothesenüberzugs 100, beschränkt. So kann das Ende des Teilabschnitts 22 wie in Fig. 3d gezeigt zwischen Außenhaut 101 und Innenauskleidung 13 eingeschoben sein, und alle drei Elemente 101, 13 und 22 können gemeinsam in einem z. B. dreilagigen Klebeabschnitt wie er für die Verbindung aus den Elemente 101, 33 und 22 als Klebeabschnitt 35 (oder einem anderen Verbindungsabschnitt) gezeigt ist, miteinander verbunden, hier verklebt, sein. Alternativ ist der Teilabschnitt 22 mit der Außenhaut 101 verbunden oder verklebt, und die Außenhaut 101 mit der Innenauskleidung 13, nicht aber unmittelbar auch der Teilabschnitt 22 mit der Innenauskleidung 13.

Die Außenhaut 101 weist hier exemplarisch über ihre gesamte Länge, jedenfalls aber in den Übergängen zwischen erstem und zweitem Abschnitt 10, 20 und/oder zwischen zweitem und drittem Abschnitt 20, 30 keine umlaufende Naht auf, womit Fertigungs- und Haltbarkeitsvorteile verbunden sein können.

Die Außenhaut 101 kann somit - zumindest in Längsrichtung - durchgehend ausgestaltet sein, aus einem ununterbrochenen Textil, einer solchen Membran, Folie, usw.

Der zweite Abschnitt 20, welcher zwischen erstem Abschnitt 10 und drittem Abschnitt 30 angeordnet ist und somit deren Verbindung darstellt, weist in manchen beliebigen Ausführungsformen, wie z. B. jener der Fig. 3d, den Teilabschnitt 22 auf, welcher aus den beiden hier mit Blick jeweils auf ihre schmale Seite gezeigten Bändern und der ihnen radial außen, also zu ihrer Ausseiten zugeordneten Außenhaut 101 besteht oder diese zumindest aufweist.

Zu erkennen ist, dass die Außenhaut 101 in dieser Ausführungsform wenigstens zwei Durchmesser und/oder Umfänge aufweist, nämlich einen ersten Durchmesser und/oder Umfang im Bereich des ersten Abschnitts 10 und einen zweiten im Bereich des zweiten Abschnitts 20, wobei der erste Durchmesser und/oder Umfang kleiner ist als der zweite.

Wenn hierin die Rede davon ist, dass der erste Durchmesser oder der Umfang kleiner ist als der zweite Durchmesser oder Umfang, so kann sich dies auf einen entspannten Zustand des Prothesenüberzugs 100 beziehen, etwa wenn dieser nicht getragen wird sondern z. B. noch im Schrank oder in der Verpackung liegt.

Der erste Durchmesser oder der erste Umfang können z. B. deshalb kleiner sein als der zweite Durchmesser bzw. Umfang, weil zur Fertigung des Prothesenüberzugs 100 weniger Material für die Außenhaut 101 im Bereich des ersten Durchmessers/Umfangs als im Bereich des zweiten Durchmesser verwendet wurde, der Umfang somit bei an sich gleicher Wandstäre geringer ist.

Der erste Durchmesser oder der erste Umfang können z. B. aber auch deshalb kleiner sein als der zweite Durchmesser bzw.

Umfang, weil bei der Fertigung des Prothesenüberzugs 100 die Außenhaut 101 im Bereich des zweiten Durchmessers oder Umfangs bleibend vorgedehnt wurde, was im Bereich des ersten Durchmessers oder Umfangs nicht der Fall war. Die Vordehnung kann eine plastische Verformung mit sich bringen, weshalb die Außenhaut 101 im Bereich des zweiten Durchmessers oder Umfangs eine geringere Wandstäre als im Bereich des ersten Durchmessers oder Umfangs haben kann.

Der erste Durchmesser oder Umfang kann aber z. B. auch deshalb kleiner sein als der zweite Durchmesser, weil bei der Fertigung des Prothesenüberzugs 100 die Außenhaut 101 im Bereich des ersten Durchmessers oder Umfangs gedoppelt oder in Wellen gelegt und so mit der Innenauskleidung 13 und/oder 33 verbunden, z. B. verklebt wurde. Auf diese Weise hätte die Außenhaut 101, würde man sie aus ihrer Verbindung oder Verklebung lösen und anschließend glattstreichen wollen, im Bereich des ersten Durchmessers einen ersten Umfang, der so groß ist wie der zweite Umfang oder es sein kann. Im verbundenen Zustand, also bei fertigem Prothesenüberzug 100, wie er z. B. in Fig. 3d gezeigt ist, kann sich die Außenhaut 101 aufgrund der Doppelung, Raffung, Wellung, usw. im ersten Abschnitt 10 und der Tatsache, dass sie im Bereich des ersten Abschnitts 10 mit der Innenauskleidung 13 und/oder 33 verbunden ist, welche von geringerem Umfang und/oder geringerem Durchmesser als die Außenhaut 101 im Bereich ihres zweiten Durchmessers ist, eben nicht frei entfalten. Ihr Durchmesser und/oder Umfang entspricht somit in etwa dem Außendurchmesser oder Außenumfang der Innenauskleidung 13. Ein solcher Fall ist in Fig. 3e gezeigt.

Die Wahl der unterschiedlichen Durchmesser oder Umfänge führt zu einer Form des Prothesenüberzugs, wie sie in Fig. 3d und Fig. 3e gezeigt ist. Auf einen engeren ersten Abschnitt 10 folgen der weitere oder breitere zweite Abschnitt 20 und der dritte Abschnitt 30, der optional wieder enger als der zweite Abschnitt 20 ist, ggf. sogar von selbem Durchmesser oder Umfang wie der erste Abschnitt 10.

Der Teilabschnitt 22 kann wie in anderen hierin offenbarten Ausführungsformen sicherstellen, dass der Abstand zwischen dem ersten Abschnitt 10 und dem dritten Abschnitt 30 (in Längsrichtung L) einen Höchstabstand oder einen gewünschten Maximalabstand zwischen erstem Abschnitt 10 und drittem Abschnitt 30 nicht überschreitet.

Dabei ist der zweite Abschnitt 20 aufgrund seines Teilabschnitts 22 vorzugsweise weniger elastisch als der erste Abschnitts 10 und/oder der dritte Abschnitt 30. Er kann optional nicht-elastisch sein, insbesondere was seinen zweiten Teilabschnitt 22 betrifft.

Was hierin für die Innenauskleidung 13 oder den ersten Abschnitt 10 ausgeführt ist, kann ungeschmälert auch für eine Innenauskleidung 33 zutreffen, welche optional an einer Innenseite der Außenhaut 101 im Bereich des dritten Abschnitts 30 in Fig. 3d gezeigt ist, und/oder für den dritten Abschnitt 30.

Anders als der erste Abschnitt 10 und/oder der dritte Abschnitt 30, weist der zweite Abschnitt 20 vorzugsweise keine Innenauskleidung, insbesondere nicht im Sinne der Innenauskleidung 13 oder 33, auf.

**Fig. 3e** zeigt einen erfindungsgemäßen Prothesenüberzug 100 in einer siebten Ausführungsform in Seitenansicht mit leichter Perspektive von oben.

Wie Fig. 3e zu entnehmen ist, weist der Prothesenüberzug 100 wie bereits jener der Fig. 3d in seinem zweiten Abschnitt 20 einen Umfang und/oder einen Durchmesser D20 auf, welcher größer ist als der Umfang bzw. der Durchmesser D10 des ersten Abschnitts, und welcher ebenfalls größer ist als der Umfang bzw. der Durchmesser D30 des dritten Abschnitts 30.

Rein optional weist der Prothesenüberzug 100 der Fig. 3e eines oder mehrere der Merkmale in beliebiger Kombination auf, welche hierin beschrieben sind.

Insbesondere kann der Prothesenüberzug 100 der Fig. 3e optional beliebige Merkmale der Ausführungsform der Fig. 3d aufweisen.

Weiter kann der Prothesenüberzug 100 der Fig. 3e oder jeder anderen Ausführungsform optional die folgenden Merkmale jeweils allein oder in beliebiger Kombination miteinander und/oder mit anderen Merkmalen wie hierin beschrieben aufweisen.

So sind die Innenauskleidung 13 in Fig. 3e, und hier exemplarisch auch die Innenauskleidung 33, auf ihrer Außenseite jeweils wellig gefertigt, bei vorzugsweise glatter Innenseite wie der Blick in das Innere I des Prothesenüberzugs 100 zeigt.

Die wellige Außenseite kann mit der Außenhaut 101 derart verklebt sein, dass sich diese in jenen Bereichen, in welchen sie mit der Innenauskleidung 13 oder 33 z. B. verklebt ist, ebenfalls in Wellen legt. Dies ist im freien Bereich, d. h. dort, wo die Außenhaut 101 nicht wie vorstehend beschrieben verklebt ist, anders. Dort kann sie wellenfrei liegen. Letzteres ist der Grund, weshalb die Außenhaut 101 im Bereich ihrer Verklebung mit einer der Innenauskleidungen 13, 33 einen geringeren Durchmesser (siehe Fig. 3e) aufweist, obwohl sie dort - im entfalteten Zustand - optional den gleichen Umfang aufweist.

Der maximale Umfang liegt im Bereich des ersten Abschnitts 10 und/oder im Bereich des dritten Abschnitts 30 bei beispielsweise 20 cm, während der maximale Umfang im Bereich des zweiten Abschnitts 20 beispielsweise 50 cm beträgt.

Wie anhand des per Strichlinie gezeigten oberen oder proximalen Teils der Innenauskleidung 33 zu erkennen ist, kann die Innenauskleidung 13 oder 33 nur teilweise mit der Außenhaut 101 verbunden oder verklebt sein. Ein Teil der Innenauskleidung 13, 33 kann frei von einer Verklebung sein.

Der optionale Teilabschnitt 22 des Fig. 3d ist hier nicht gezeigt und auch nicht angedeutet.

In einigen Ausführungsformen ist die Außenhaut 101 der erste Teilabschnitt 21. Was hierin für die Außenhaut 101 ausgeführt ist, kann ungeschmälert für den ersten Teilabschnitt 21 gelten, und in manchen Ausführungsformen gilt dies umgekehrt ebenfalls.

**Fig. 4** zeigt einen erfindungsgemäßen Prothesenüberzug 100 in einer achten Ausführungsform.

Der Prothesenüberzug 100 entspricht jener der Fig. 3, außer dass es hier nur einen zweiten Teilabschnitt 22 gibt, der sich über 180° des Umfangs des Prothesenüberzugs 100 erstreckt.

Der erste Teilabschnitt 21 kann sich, wie in Fig. 4 exemplarisch gezeigt, optional mit dem zweiten Teilabschnitt 22 zu 360° ergänzen.

Der erste Teilabschnitt 21 kann den Umfang der Extremität oder des Prothesenüberzugs 100 optional zu 360° abdecken. Der zweite Teilabschnitt 22 kann dem ersten Teilabschnitt 21 optional an dessen Innen- und/oder Außenseite auf- oder anliegen.

Der erste Teilabschnitt 21 kann, wie in Fig. 4 exemplarisch gezeigt, 180° des Umfangs des Prothesenüberzugs 100 ausmachen oder bedecken. Er kann alternativ aber auch breiter als in Fig. 4 gezeigt sein. So kann er z. B. bis zu 240° oder mehr des Umfangs des Prothesenüberzugs 100 ausmachen.

Der erste Teilabschnitt 21 kann in jeder erfindungsgemäßen Ausführungsform zwischen 40 % und 70 % des Umfangs bedecken, besonders bevorzugt einen Teilumfang, der zwischen 55 % und 65 % des Umfangs ausmacht, ganz besonders bevorzugt 60 % des Umfangs.

Vorstehende Prozentangaben können sich optional auf den entspannten Zustand des Prothesenüberzugs 100, etwa vor Gebrauch, z. B. in der Versandverpackung, oder auf einen Gebrauchszustand, etwa an der Extremität, beziehen.

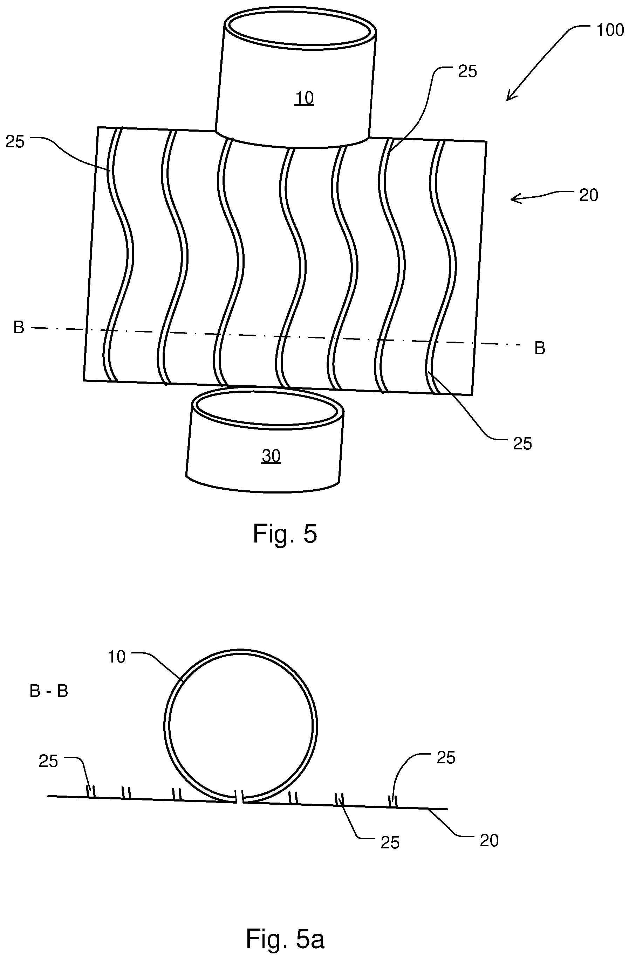

**Fig. 5** zeigt einen erfindungsgemäßen Prothesenüberzug 100 in einer neunten Ausführungsform in einem Teilschnitt.

Geschnitten und aufgeklappt bzw. abgewickelt ist nur der zweite Abschnitt 20, nicht aber auch der erste Abschnitt 10 und der dritte Abschnitt 30. Der Blick auf die Zeichenebene der Fig. 5 zeigt die Innenseite des zweiten Abschnitts 20, die im Gebrauch des Prothesenüberzugs 100 der Haut des Patienten anliegt oder dieser jedenfalls zugewandt wäre.

Zu erkennen ist eine erhabene Struktur 25, die sich von der Innenseite des zweiten Abschnitt 20 weg und im Gebrauch des Prothesenüberzugs 100 radial in deren Inneres erstreckt.

Die Struktur 25 kann bevorzugt in einer Längsrichtung des Prothesenüberzugs 100 verlaufen. Sie dient dazu, ein vollkommen planes Anliegen des zweiten Abschnitts 20 am Körper des Patienten zu vermeiden. Begünstigt durch den Abstand, den die Struktur 25 zwischen der Innenseite des zweiten Abschnitts 20 und dem Körper des Patienten (z. B. der Haut) offenhält, kann ein Unterdruck zwischen der Innenseite des zweiten Abschnitts 20 und dem Körper des Patienten mit geeigneten Unterdruckvorrichtungen ohne relevante Nischenbildung erzeugt werden.

**Fig. 5a** zeigt den einen Schnitt durch den erfindungsgemäßen Prothesenüberzug 100 der Fig. 5 entlang einer Linie B-B der Fig. 5.

**Bezugszeichenliste**

100    Prothesenüberzug

101    Außenhaut

10    erster Abschnitt

11    Bereich

13    Innenauskleidung

20    zweiter Abschnitt

21    erster Teilabschnitt

| | |
|---|---|
| 22 | zweiter Teilabschnitt |
| 23 | Doppelungen, gedoppelter Bereich |
| 25 | erhabene Struktur |
| 30 | dritter Abschnitt |
| 31 | Überstand |
| 32 | Befestigungsabschnitt |
| 33 | Innenauskleidung |
| 35 | Klebebereich |
| 40 | Schnittlinie |
| 50 | Zeichenlineal |
| D10 | Durchmesser des ersten Abschnitts |
| D20 | Durchmesser des zweiten Abschnitts |
| D30 | Durchmesser des dritten Abschnitts |
| B-B | Schnittlinie |
| I | Inneres des Prothesenüberzugs |
| L | Längsrichtung |

**Patentansprüche**

1. Prothesenüberzug (100) mit einem geschlossenen Querschnitt,

   wobei der Prothesenüberzug (100) entlang seiner Längserstreckung wenigstens einen ersten Abschnitt (10), einen zweiten Abschnitt (20) und einen dritten Abschnitt (30) aufweist,
   wobei der zweite Abschnitt (20) einen größeren Umfang oder Durchmesser (D20) aufweist als sowohl der erste Abschnitt (10) als auch der dritte Abschnitt (30) und/oder wobei der zweite Abschnitt (20) aus einem Material besteht oder ein solches aufweist, welches einen größeren Elastizitätsmodul aufweist als eines oder mehrere der Materialien des ersten Abschnitts (10) und/oder des dritten Abschnitts (30); und
   wobei der zweite Abschnitt (20) zwischen dem ersten Abschnitt (10) und dem dritten Abschnitt (30) angeordnet ist, **dadurch gekennzeichnet, dass**
   der zweite Abschnitt (20) in einem Verbindungs- oder Befestigungsabschnitt (32) endet, in welchem der zweite Abschnitt (20) mit dem dritten Abschnitt (30) verbunden ist, wobei der dritte Abschnitt (30) über das untere Ende des zweiten Abschnitts (20) über den Befestigungsabschnitt (32) in Richtung zum ersten Abschnitt (10) als Überstand (31) übersteht, und dass der Überstand (31) des dritten Abschnitts (30) ganz oder abschnittsweise nicht mit dem zweiten Abschnitt (20) verbunden ist.

2. Prothesenüberzug (100) nach Anspruch 1, mit einer Schicht oder Außenhaut (101), welche sich durchgehend über den ersten Abschnitt (10), den zweiten Abschnitt (20) und den dritten Abschnitt (30) erstreckt, wobei die Schicht oder Außenhaut (101) im ersten Abschnitt (10) und/oder im dritten Abschnitt (30) mit einer Innenauskleidung (13, 33), vorzugsweise direkt oder indirekt und/oder in radialer Richtung, verbunden ist, nicht jedoch im zweiten Abschnitt (20).

3. Prothesenüberzug (100) nach Anspruch 1 oder 2, wobei der erste Abschnitt (10), der zweite Abschnitt (20) und/oder der dritte Abschnitt (30) ein luftdichtes Material aufweist oder hieraus besteht.

**4.** Prothesenüberzug (100) nach einem der Ansprüche 2 bis 3, wenn abhängig von Anspruch 2, wobei die erste Innenauskleidung (13) und/oder die zweite Innenauskleidung (33) aus Silikon besteht oder Silikon aufweist.

**5.** Prothesenüberzug (100) nach einem der Ansprüche 2 bis 4, wenn abhängig von Anspruch 2, wobei ein erster Teilabschnitt (21) des zweiten Abschnitts (20) oder die Außenhaut (101) in einer Umfangsrichtung nicht elastisch ist.

**6.** Prothesenüberzug (100) nach einem der vorangegangenen Ansprüche, wobei der erste Abschnitt (10) und/oder der dritte Abschnitt (30) elastisch sind.

**7.** Prothesenüberzug (100) nach einem der vorangegangenen Ansprüche, wobei der zweite Abschnitt (20) an seiner Innenseite wenigstens eine bandförmige Struktur als zweiten Teilabschnitt (22) aufweist.

**8.** Prothese, insbesondere Beinprothese oder Knieprothese, die zumindest teilweise bedeckt oder überdeckt ist durch einen Prothesenüberzug (100) nach einem der vorangegangenen Ansprüche.

**9.** Set mit einer Prothese und mit einem Prothesenüberzug (100) nach einem der Ansprüche 1 bis 7.

**Claims**

**1.** A prosthesis covering (100) having a closed cross-section,

the prosthesis covering (100) along the longitudinal extension thereof comprising at least a first section (10), a second section (20) and a third section (30),
the second section (20) having a larger circumference or diameter (D20) than both the first section (10) and the third section (30) and/or the second section (20) being made of or comprising a material that has a larger modulus of elasticity than one or more of the materials of the first section (10) and/or of the third section (30); and
the second section (20) being arranged between the first section (10) and the third section (30), **characterized in that**
the second section (20) ends in a connecting or fastening section (32) in which the second section (20) is connected to the third section (30), the third section (30) protruding beyond the lower end of the second section (20), beyond the fastening section (32), toward the first section (10) in the form of a protrusion (31), and **in that** the protrusion (31) of the third section (30), in whole or in sections, is not connected to the second section (20).

**2.** The prosthesis covering (100) according to claim 1, comprising a layer or outer skin (101), which extends continuously over the first section (10), the second section (20) and the third section (30), wherein the layer or outer skin (101) in the first section (10) and/or in the third section (30), but not in the second section (20), is connected to an interior lining (13, 33), preferably directly or indirectly and/or in the radial direction.

**3.** The prosthesis covering (100) according to claim 1 or 2, wherein the first section (10), the second section (20) and/or the third section (30) comprise an air-tight material or are made thereof.

**4.** The prosthesis covering (100) according to any one of claims 2 to 3, when dependent on claim 2, wherein the first interior lining (13) and/or the second interior lining (33) are made of silicone or comprise silicone.

**5.** The prosthesis covering (100) according to any one of claims 2 to 4, when dependent on claim 2, wherein a first sub-section (21) of the second section (20) or the outer skin (101) is not elastic in a circumferential direction.

**6.** The prosthesis covering (100) according to any one of the preceding claims, wherein the first section (10) and/or the third section (30) are elastic.

**7.** The prosthesis covering (100) according to any one of the preceding claims, wherein the second section (20), on the inner side thereof, has at least one ribbon-shaped structure serving as a second sub-section (22).

**8.** A prosthesis, in particular leg prosthesis or knee prosthesis, which at least partly covers or is blanketed by a prosthesis covering (100) according to any one of the preceding claims.

9. A set comprising a prosthesis and a prosthesis covering (100) according to any one of claims 1 to 7.


**Revendications**

1. Revêtement de prothèse (100) avec une section transversale fermée,

   dans lequel le revêtement de prothèse (100) présente, le long de son extension longitudinale, au moins une première section (10), une deuxième section (20) et une troisième section (30),
   dans lequel la deuxième section (20) présente une circonférence ou un diamètre (D20) plus grand qu'à la fois la première section (10) et la troisième section (30) et/ou dans lequel la deuxième section (20) est constituée de ou comprend un matériau, lequel présente un module d'élasticité plus grand qu'un ou plusieurs des matériaux de la première section (10) et/ou de la troisième section (30) ; et
   dans lequel la deuxième section (20) est disposée entre la première section (10) et la troisième section (30),
   **caractérisé en ce que**
   la deuxième section (20) se termine dans une section de liaison ou de fixation (32) dans laquelle la deuxième section (20) est reliée à la troisième section (30), dans lequel la troisième section (30) dépasse de l'extrémité inférieure de la deuxième section (20) au-delà de la section de fixation (32) en direction de la première section (10) sous la forme d'une saillie (31), et que la saillie (31) de la troisième section (30) n'est pas reliée à la deuxième section (20) en totalité ou par sections.

2. Revêtement de prothèse (100) selon la revendication 1, avec une couche ou peau extérieure (101), laquelle s'étend de manière continue sur la première section (10), la deuxième section (20) et la troisième section (30), dans lequel la couche ou peau extérieure (101) est reliée à un revêtement intérieur (13, 33) dans la première section (10) et/ou dans la troisième section (30), de préférence directement ou indirectement et/ou dans la direction radiale, mais pas dans la deuxième section (20).

3. Revêtement de prothèse (100) selon la revendication 1 ou 2, dans lequel la première section (10), la deuxième section (20) et/ou la troisième section (30) comprend un matériau étanche à l'air ou en est constituée.

4. Revêtement de prothèse (100) selon l'une quelconque des revendications 2 à 3, lorsqu'elle dépend de la revendication 2,
   dans lequel le premier revêtement intérieur (13) et/ou le deuxième revêtement intérieur (33) est constitué de silicone ou comprend du silicone.

5. Revêtement de prothèse (100) selon l'une quelconque des revendications 2 à 4, lorsqu'elle dépend de la revendication 2,
   dans lequel une première section partielle (21) de la deuxième section (20) ou la peau extérieure (101) est non élastique dans une direction circonférentielle.

6. Revêtement de prothèse (100) selon l'une quelconque des revendications précédentes, dans lequel la première section (10) et/ou la troisième section (30) sont élastiques.

7. Revêtement de prothèse (100) selon l'une quelconque des revendications précédentes, dans lequel la deuxième section (20) présente sur sa face intérieure au moins une structure en forme de bande comme deuxième section partielle (22).

8. Prothèse, en particulier prothèse de jambe ou prothèse de genou, qui est au moins partiellement recouverte ou couverte par un revêtement de prothèse (100) selon l'une quelconque des revendications précédentes.

9. Ensemble avec une prothèse et avec un revêtement de prothèse (100) selon l'une quelconque des revendications 1 à 7.

Fig. 1

Fig. 2

Fig. 2a

Fig. 3

Fig. 3a

Fig. 3b

Fig. 3c

Fig. 3d

Fig. 3e

Fig. 4

100

25

10

25

20

25

25

B ─ · ─ · ─ · ─ · ─ · ─ · ─ · ─ · ─ · ─ · ─ B

25

30

## Fig. 5

B - B

10

25

25

25

20

25

20

## Fig. 5a

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019110543 A1